# EUROPEAN PATENT SPECIFICATION

(11) **EP 2 204 126 B1**
(45) Date of publication and mention of the grant of the patent: **23.11.2011**
(21) Application number: 10160890.9
(22) Date of filing: 11.10.2007
(51) Int. Cl.: A61B 17/28, A61B 17/00, A61B 19/00

(54) **Manipulator**
Manipulator
Manipulateur

(30) Priority: 13.10.2006 JP 2006280349; 13.10.2006 JP 2006280355; 13.10.2006 JP 2006280361
(43) Date of publication of application: 07.07.2010
(62) Divisional of application: 07118293.5
(73) Proprietor: Terumo Kabushiki Kaisha, Tokyo 151-0072 (JP); Kabushiki Kaisha Toshiba, Tokyo 105-8001 (JP)
(72) Inventor: Omori, Shigeru, Ashigarakami-gun Kanagawa 259-0151 (JP); Jinno, Makoto, Fujinomiya-shi Shizuoka 418-0015 (JP); Sunaoshi, Takamitsu, Minato-ku Tokyo 105-8001 (JP)
(74) Representative: TBK

(56) References cited:
- DE-A1- 4 136 861
- US-A- 5 582 617
- US-A1- 2004 052 016
- US-B1- 6 352 477

## Description

### BACKGROUND OF THE INVENTION

### Field of the Invention:

The present invention relates to a manipulator having a hand-gripped operating unit, a connector extending from the operating unit, and a working unit angularly movable when the operating unit is operated.

### Description of the Related Art:

In endoscopic surgery (also called laparoscopic surgery), it is customary to form a plurality of incisions in the body surface of the patient, insert trocars (tubular instruments) into the respective incisions as forceps instrument passage ports, and to introduce the tip ends of forceps instruments having shafts through the respective trocars into a body cavity, to perform a surgical operation on an affected part of the body. Working units such as a gripper for gripping living tissue, scissors, an electrosurgical knife blade, etc., are mounted on the tip ends of the forceps instruments.

An endoscopic surgical operation performed with forceps instruments requires the surgeon to be trained in advance, because the working space in the body cavity is small and the forceps instruments need to be operated using the trocars as fulcrums. Since the forceps instruments that have been used heretofore have no joints in the working unit at the tip end thereof, the forceps instruments have a small degree of freedom, and the working unit can be operated only on an extension of the shaft. Therefore, cases that can be handled under usual endoscopic surgery training practices are limited to a certain range, and surgeons need to be highly trained and quite skilled in order to be able to perform endoscopic surgery in various other cases outside of the limited range.

Attempts have heretofore been made to improve conventional forceps instruments, in order to develop a forceps instrument having a plurality of joints in the working unit thereof (see, for example, Japanese Laid-Open Patent Publication JP 2002-102248A). Such developed forceps instruments, which may also be referred to as manipulators, are free of the limitations and difficulties inherent in conventional forceps instruments, can be operated using easy techniques, and can be applied to a wide variety of cases.

Another developed endoscopic surgical system is based on robotic technology, which is introduced into a working unit having a plurality of joints. Specifically, the endoscopic surgical system incorporates a master-slave remote control system, developed in the field of industrial robots. The surgeon is seated at a working station, i.e., a master console, spaced from a surgical bed on which the patient lies, wherein the surgeon moves an operating handle in order to perform operations while watching a display monitor. The surgical bed is equipped with a robotic arm, i.e., a slave manipulator, on which a forceps instrument is mounted. The forceps instrument includes a shaft inserted through a trocar into the body cavity of the patient. When the surgeon moves the operating handle, motion is converted into electric signals, which are supplied to a computer in order to move the working unit on the distal end of the forceps instrument, which reaches the affected part of the patient.

Since the working unit on the tip end of the forceps instrument has a plurality of joints, the working unit can move with a high degree of freedom, reflecting wrist motions of the surgeon. Because of the robotic arm, the tip end of the forceps instrument can be positioned within the body cavity by the operating handle. Therefore, limitations imposed by using a trocar acting as a fulcrum are eliminated.

However, it has been pointed out that surgical operations performed with a master-slave robot suffer from the following problems: 1. Since the slave manipulator constructed as a robotic arm needs to be positioned and fixed in advance, it takes time to prepare for surgery. 2. Since it is also necessary to position the robotic arm in advance for each surgical case, it is burdensome to draw up operation protocols, and such procedures are not applicable to certain surgical cases. 3. If unexpected trouble happens during the surgical operation, and the operation needs to be changed to a thoracotomy or a laparotomy, then since it is time-consuming to remove the robotic arm, the duration of the operation increases, resulting in a highly invasive situation for the patient. 4. The overall endoscopic surgical system is complex in structure, large in scale, and highly expensive to manufacture.

Efforts have been made to dispense with such a master-slave system, and improve the forceps instrument so as to act as a manipulator having a compact working unit on its tip end and a higher degree of freedom, whereby the forceps instrument itself is applicable to a variety of surgical cases and is easily operable.

For developing such a manipulator, it is important to develop not only a working unit on the tip end of the manipulator, but also to provide an operating means for use in combination therewith. This is because the increased degree of freedom of the working unit results in an increased number of input members, which makes the working unit complex to operate, for example. Further, it is time-consuming for the surgeon to confirm operation of the working unit in yawing and rolling directions, and also in positive and negative directions, so that the manipulator cannot be operated using easy techniques.

In particular, there have been demands for a manipulator, which is capable of rolling the working unit easily, as well as an operating means for such a manipulator, for use in endoscopic surgical operations in which suture knot tying is an indispensable task.

In particular, endoscopic surgery often requires fine manipulations in long hours. Accordingly, there have been demands for a manipulator which can be operated easily and intuitively even during a complex joint motion of a working unit of the manipulator.

When the operating unit needs to be halted without operation, a user releases the hand from the input unit to maintain the input unit at a neutral position. However, if the input unit is slightly displaced from the neutral position, the operating unit undesiredly continues to move in one of opposite directions at a low speed. Also, even if the input unit is maintained at the neutral position, when an unintentional slight touch on the input unit results in moving the operating unit, a user cannot operate it easily. Stated otherwise, when response sensitivity of the manipulator is too high, the manipulator is not easy for the user to operate.

Document DE 41 36 861 Al discloses a manipulator having the features of the preamble of claim 1. This manipulator has a working unit disposed on a distal end of a connector. In the connector, a wire which is torsion proof, is used for transmitting a torsion force to the working unit.

Document US 5 582 617 A discloses a manipulator having a working unit disposed on a distal end of a connector. In the connector, a wire is used for transmitting a pushing/pulling force to the working unit. In a modification, a rotating force is transmitted to the working unit.

### SUMMARY OF THE INVENTION

It is the object of the present invention to provide a manipulator, which is capable of rolling a working unit with ease, while allowing a user to learn how to operate the manipulator within a short period of time during a training process.

It is a further aim of the present invention is to provide a manipulator, which is capable of tying a suture knot in a living tissue inside a deep and narrow area within a body cavity.

It is a further aim of the present invention to provide a manipulator in which a user can control easily and intuitively a complex motion of a working unit.

It is a further aim of the present invention to provide a manipulator which can be operated intuitively and which has a high operability. The above object is solved by a manipulator having the features of claim 1.

A manipulator which is not claimed comprises an operating unit including a grip handle to be gripped by a hand, a first input unit mounted on the operating unit and operable by a finger, a connector extending from the operating unit, and a working unit disposed on a distal end of the connector and including a rotating mechanism angularly movable about an axis along which a distal end of the working unit extends, based on operation of the first input unit, wherein the first input unit comprises a rotary member including at least two finger holders disposed at left and right positions, respectively, on each side of a central line of the rotary member as viewed in front elevation.

The first input unit as viewed in front elevation is defined as the first input unit viewed along an axis of rotation of the first input unit. The left and right positions are defined as positions recognized when the grip handle is oriented downwardly.

When the user applies a thumb, for example, to either one of the finger holders and pushes the finger holder upwardly, the first input unit is turned, so as to turn the rotating mechanism about an axis in the same direction as the first input unit. Since the rotating mechanism and the first input unit operate substantially in the same manner, the user can operate the working unit easily and intuitively. Furthermore, the first input unit allows the rotating mechanism to turn in opposite directions. Therefore, the first input unit does not require an increased number of input members, and hence the operating unit is simple in structure and easy to operate. The two finger holders allow the user to confirm the position of the first input unit through tactile sensation, without the need to observe the hand, and also allow the user to operate the first input unit under a light force without slippage.

The operating unit may include a second input unit disposed within an angularly movable range of the two finger holders and operable by the finger, so as to be tilted in horizontally pushed directions. Also, the working unit may include a pivot axis about which the working unit is angularly movable and which is non-parallel to an axis that extends from a proximal end of the connector to the distal end thereof, based on operation of the second input unit.

The first input unit may have an annular shape when viewed in front elevation, wherein the operating unit may include a second input unit, disposed within the first input unit and operable by the finger so as to be tilted in vertically pushed directions. Also, the working unit may include a pivot axis about which the working unit is angularly movable and which is non-parallel to an axis that extends from a proximal end of the connector to the distal end thereof, based on operation of the second input unit.

Because the first input unit and the second input unit are independent of each other, the user finds it easy to turn the rotating mechanism and the pivot axis separately from each other, and the user does not become confused when operating the first input unit and the second input unit. The second input unit is disposed inside of a range in which the two finger holders are angularly movable. Therefore, the operating unit consists of a compact input means, which allows the finger to move over a short distance, while also allowing the first and second input units to be operated with a single finger. The second input unit allows the pivot axis to turn in opposite directions. Therefore, the second input unit does not require an increased number of input members, but rather, is simple in structure and easy to operate.

The first input unit may be angularly movable within a range of from ± 5° to ± 20°. The first input unit may have a center of rotation spaced from each of the finger holders by a distance within a range of from 10 mm to 25 mm. An angle formed between an axis along which the connector extends and a rotational plane of the first input unit may be within a range of from 35° to 55°. With these numerical settings, the first input unit is suitable for naturally positioning the thumb, and corresponds to the movable range of the thumb, thereby allowing the thumb to operate the first input unit easily.

The first input unit, when viewed in front elevation, may have a vertical central line aligned with an axis along which the connector extends. With this arrangement, the user is able to feel as though the first input unit and the rotating mechanism operate concentrically and in a direct relationship with each other, and thus the user finds it easy to operate the manipulator.

The first input unit may have an annular shape when viewed in front elevation. The annular shape of the first input unit allows the user to visually recognize the first input unit easily, as an input means that is capable of twisted motion. The user can easily learn and will not quickly forget how to utilize the first input unit.

The two finger holders may have a center of rotation disposed in a position shifted from an axis along which the connector extends, in a direction opposite to the direction in which the grip handle extends. Therefore, when the manipulator is turned about a trocar, the manipulator can easily be operated simply by twisting the wrist. However, in this case, the wrist is not turned along a large arcuate path.

The center of rotation of the two finger holders may be spaced from the axis along which the connector extends, by a distance lying within a range of from 20 mm to 50 mm.

When the user applies the thumb to either one of the finger holders and pushes the finger holder upwardly in order to turn the first input unit in one direction, the rotating mechanism on the distal end is turned in the same direction. Therefore, the rolling mechanism and the first input unit both turn in the same direction, so that the user can operate the working unit easily and intuitively. Since the working unit can be operated with ease, the user can learn how to operate the working unit during a training process within a short period of time.

The two finger holders allow the user to confirm positioning of the first input unit through tactile sensation, without the need to look at the hand, while also allowing the user to operate the first input unit under a light force without slippage.

Further, the manipulator is capable of tying a suture knot in a living tissue inside a deep and narrow area within a body cavity.

A further manipulator which is not claimed comprises an operating unit including a grip handle to be gripped by a hand, a composite input unit mounted on the operating unit and having a first input unit and a second input unit, each operable by a finger, a connector extending from the operating unit, and a working unit disposed on a distal end of the connector, wherein the working unit includes a rotating mechanism angularly movable about an axis along which the distal end of the working unit extends, based on operation of the first input unit and a pivot axis about which the working unit is angularly movable and which is non-parallel to an axis that extends from a proximal end of the connector to the distal end thereof, based on operation of the second input unit, the first input unit comprises a rotary member for supplying an operation command to the rotating mechanism, the second input unit is tilted in horizontally pushed directions in order to supply an operation command with respect to the rotation of the pivot axis, and the first input unit surrounds the second input unit at least with respect to a tilted direction of the second input unit and is coaxially disposed with respect to a center of the second input unit, as viewed from the front of the composite input unit.

The composite input unit as viewed from the front thereof is defined as the composite input unit viewed along an axis of rotation of the first input unit. The left and right positions are defined as positions recognized when the grip handle is oriented downwardly.

In the composite input unit of the manipulator, since the first input unit and the second input unit are arranged concentratedly on the composite input unit, the first input unit and the second input unit can be operated by moving a finger within a small range. Further, the complex motion of the first input unit and the second input unit can be easily performed by one finger. The term "complex motion" here includes not only two or more motions that are simultaneously performed, but also two or more motions that are alternately performed at short intervals.

In the composite input unit, the rolling motion of the first input unit corresponds to the rolling motion of the rolling mechanism, and the tilting motion of the second input unit corresponds to the tilting motion of the pivot axis. Thus, the user can operate the working unit easily and intuitively.

Also, since the first input unit and the second input unit are provided separately, operations of the rolling mechanism and the pivot axis can be correctly performed without confusion thereof.

The first input unit and the second input unit allow the rotating mechanism and the pivot axis to turn in opposite directions, respectively. Therefore, the first input unit and the second input unit do not require an increased number of input members, and hence the operating unit is simple in structure and easy to operate.

The second input unit may have a width ranging from 10 mm to 25 mm, in a direction perpendicular to the tilted direction of the second input unit, as viewed from the front of the composite input unit. With these numerical settings, the second input unit is suitable for naturally positioning the thumb, and corresponds to a naturally movable range of the thumb, thereby allowing the thumb to operate the second input unit easily.

The rotary member may have an annular shape, when viewed in a direction along which the rotational axis of the rotary member extends. The annular shape of the rotary member allows the user to visually recognize the first input unit easily, as an input means that is capable of twisted motion. The user can easily learn and will not quickly forget how to utilize the first input unit.

The working unit may further comprise a third angularly moving mechanism other than the rotating mechanism and the pivot axis, the composite input unit may further comprise a third input unit operable by a finger and vertically tiltable in order to supply an operation command to the third angularly moving mechanism, the second input unit and the third input unit may be integrally formed with each other, and the first input unit may surround the second input unit and the third input unit, as viewed from the front of the composite input unit.

In the manipulator, since the first input unit and the second input unit are arranged concentratedly on the composite input unit, the first input unit and the second input unit can be operated by moving a finger within a small range. Further, the complex motion of the first input unit and the second input unit can be easily performed by one finger.

Also, in the composite input unit, the rolling motion of the first input unit corresponds to the rolling motion of the rolling mechanism, and the tilting motion of the second input unit corresponds to the tilting motion of the pivot axis. Thus, the user can operate the working unit 12 easily and intuitively.

The first input unit surrounds the second input unit with respect to at least the tilted direction, and the first input unit and the second input unit are disposed in a well-balanced fashion, in coaxial relation to the center point of the second input unit. The first input unit and the second input unit thus can be operated highly effectively and smoothly, allowing the user easily to learn how to use them.

In the composite input unit, the user can easily confirm the position of the first input unit and the second input unit through tactile sensation, without looking at the hand, and can operate them highly effectively and smoothly. Thus, the above composite input unit may appropriately be applied to perform fine techniques for long hours.

Further, the working unit may further comprise a third angularly moving mechanism other than the rotating mechanism and the pivot axis, the composite input unit may further comprise a third input unit operable by a finger and vertically tiltable in order to supply an operation command to the third angularly moving mechanism, the second input unit and the third input unit may be integrally formed with each other, and the first input unit may surround the second input unit and the third input unit, as viewed from the front of the composite input unit.

A manipulator according to the present invention comprises an operating unit including a grip handle to be gripped by a hand, a input unit mounted on the operating unit and operable in either direction with reference to a neutral position, a first detecting unit for detecting an operating amount of the input unit in one direction of the opposite directions, a second detecting unit for detecting an operating amount of the input unit in another direction of the opposite directions, a connector extending from the operating unit, and a working unit disposed on a distal end of the connector and movable in either direction based on a signal detected by the first detecting unit and the second detecting unit, wherein the first detecting unit and the second detecting unit are arranged such that an operating amount of the input unit is not detected in a range of a certain dead zone with reference to the neutral position.

As described above, the first detecting unit and the second detecting unit are arranged such that neither the first detecting unit nor the second detecting unit detects an operating amount of the input unit within a range of a certain dead zone with reference to the neutral position, which results in the presence of play in operation of the input unit. Accordingly, such play prevents a useless motion of the operating unit and an excessive reaction of the operating unit by slightly touching the input unit, thereby obtaining a high operability. Further, the operating unit is operated in either direction, in response to operations of the input unit in either direction with reference to the neutral position, respectively, thereby enabling a user to operate the manipulator intuitively.

Incidentally, an operating amount means not only a displacement amount of the input unit, but also a pressing amount etc. of the input unit.

With this arrangement, the working unit comprises a rotating mechanism angularly movable about an axis along which the distal end of the working unit extends, based on operation of the input unit, the input unit may comprise a rolling member for giving an operation command to the rotating mechanism, and the first detecting unit and the second detecting unit may be arranged such that an operating amount of the input unit is not detected within the range of the certain dead zone ranging from ± 1° to ± 8° with reference to the neutral position, thereby obtaining a higher operability.

Also, the working unit may include a pivot axis about which the working unit is angularly movable and which is non-parallel to an axis that extends from a proximal end of the connector to the distal end thereof, the input unit may comprise a tilting member tilting in horizontally pushed directions, in order to supply an operation command to the pivot axis, the first and second detecting units may face right and left portions of an end surface of the tilting member respectively, with gaps therebetween, and a distance between the first detecting unit and the second detecting unit may be set to 2 mm through 8 mm, thereby obtaining a higher operability. Further, since the first detecting unit and the second detecting unit are spaced away from each other, when the input unit is pushed down without a tilt motion, the operating unit is not moved unintentionally.

Still further, the working unit may include a first rotational axis and a second rotational axis, the input unit may comprise a tilting member for tilting in horizontally pushed directions so as to supply an operation command to the first rotational axis and for tilting in vertically pushed directions so as to supply an operation command to the second rotational axis, two sets of the first and second detecting units may be provided as a first set for detecting a horizontal tilt of the input unit and a second set for detecting a vertical tilt of the input unit, and the first set of the first and second detecting units may face right and left portions of an end surface of the tilting member with gaps therebetween, and the second set of the first and second detecting units may face upper and lower portions of the end surface of the tilting member with gaps therebetween.

According to the manipulator, the first detecting unit and the second detecting unit are arranged such that neither the first detecting unit nor the second detecting unit detects an operating amount of the input unit within a range of a certain dead zone with reference to the neutral position, which results in the presence of play in operation of the input unit. Accordingly, such play prevents a useless motion of the operating unit and an excessive reaction of the operating unit by slightly touching the input unit, thereby obtaining a high operability. Further, the operating unit is operated in either direction, in response to operations of the input unit in either direction with reference to the neutral position, respectively, thereby enabling a user to operate the manipulator intuitively.

The above and other objects, features, and advantages of the present invention will become more apparent from the following description when taken in conjunction with the accompanying drawings in which preferred embodiments of the present invention are shown by way of illustrative example.

### BRIEF DESCRIPTION OF THE DRAWINGS

FIG. 1 is a perspective view, as seen obliquely from a front end, of a manipulator according to an embodiment of the present invention;
FIG. 2 is a perspective view, shown obliquely from a rear end thereof, of the manipulator according to the embodiment of the present invention;
FIG. 3 is an exploded perspective view of a working unit of the manipulator;
FIG. 4 is a perspective view of the manipulator, which is tilted at 90° ;
FIG. 5 is an exploded perspective view of a composite input unit of the manipulator;
FIG. 6 is a rear elevational view of a base body and a shuttle ring;
FIG. 7 is a perspective view, shown obliquely from a rear end thereof, of a rubber pad;
FIG. 8 is a sectional side elevational view of the rubber pad in a neutral state, together with peripheral parts thereof;
FIG. 9 is a sectional side elevational view of the rubber pad, in a state in which a pad is operated, together with peripheral parts thereof;
FIG. 10 is a graph showing a pressing force on a pad, which is detected by a tilt-detecting pressure-sensitive sensor;
FIG. 11 is a sectional side elevational view of the shuttle ring in a neutral state, together with peripheral parts thereof;
FIG. 12 is a sectional side elevational view of the shuttle ring, in a state in which a knob is operated, together with peripheral parts thereof;
FIG. 13 is a side elevational view of the manipulator; FIG. 14 is a plan view of the manipulator, as viewed along the rotational axis of the shuttle ring;
FIG. 15 is a view showing a surface region of the composite input unit;
FIG. 16A is a perspective view of a shuttle ring according to a first modification of the present invention;
FIG. 16B is a perspective view of a shuttle ring according to a second modification of the present invention;
FIG. 17 is a sectional side elevational view of a shuttle ring according to a third modification of the present invention, and peripheral parts thereof;
FIG. 18A is a perspective view of a rubber pad having a pad according to a first modification;
FIG. 18B is a perspective view of a rubber pad having a pad according to a second modification;
FIG. 18C is a perspective view of a rubber pad having a pad according to a third modification;
FIG. 19A is a plan view of four tilt-detecting pressure-sensitive sensors arranged in a crisscross pattern;
FIG. 19B is a plan view of four tilt-detecting pressure-sensitive sensors arranged in an annular pattern;
FIG. 20 is a view showing a surface region of a composite input unit incorporating the rubber pad having the pad according to the third modification in FIG. 18C;
FIG. 21 is a perspective view of a manipulator with a composite input unit being disposed in an upwardly shifted position;
FIG. 22 is a side elevational view of the manipulator with the composite input unit being disposed in the upwardly shifted position;
FIG. 23 is a perspective view of a working unit according to a modification;
FIG. 24 is a view showing the manner in which a gripper holding a curved needle is tilted and placed near a dorsal vein complex;
FIG. 25 is a view showing the manner in which the curved needle held by the gripper is inserted into a rear surface of the dorsal vein complex;
FIG. 26A is a view showing an initial phase of a needle turning process based on operation of the shuttle ring;
FIG. 26B is a view showing a middle phase of the needle turning process based on operation of the shuttle ring; and
FIG. 26C is a view showing a final phase of the needle turning process based on operation of the shuttle ring.

### DESCRIPTION OF THE PREFERRED EMBODIMENTS

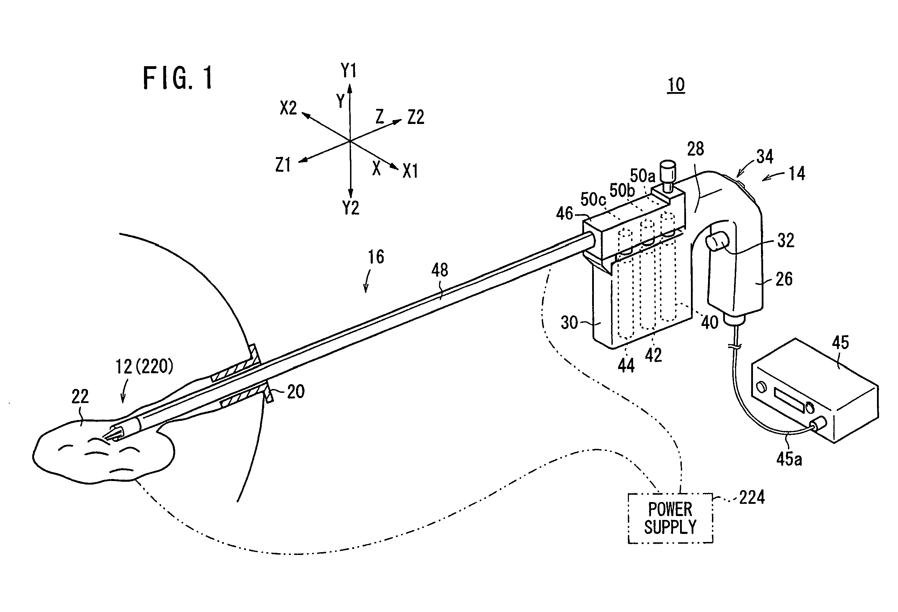

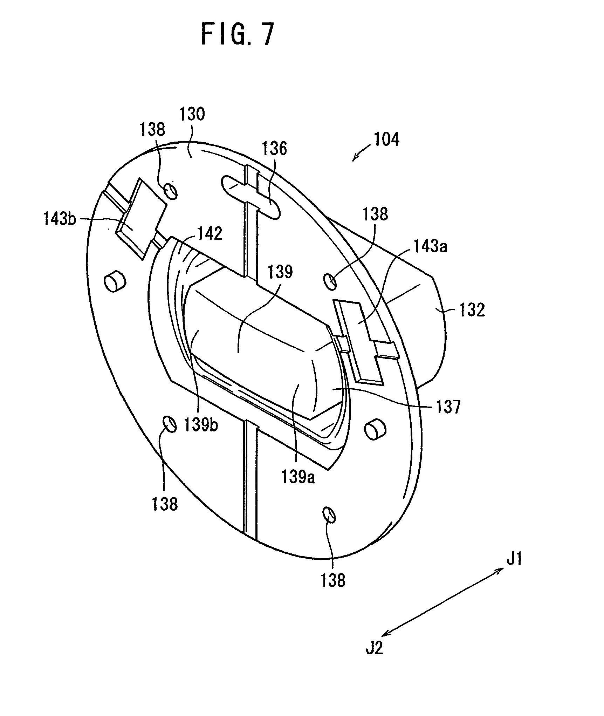

Manipulators according to preferred embodiments of the present invention shall be described below with reference to FIGS. 1 through 23. A manipulator 10 (see FIG. 1) according to an embodiment of the present invention comprises a medical manipulator for use in endoscopic surgical operations or the like.

As shown in FIG. 1, the manipulator 10 includes a working unit 12 on a tip end thereof, for gripping a portion of a living tissue, a curved needle, or the like. The manipulator 10 usually is referred to as a gripping forceps or a needle driver (needle holder).

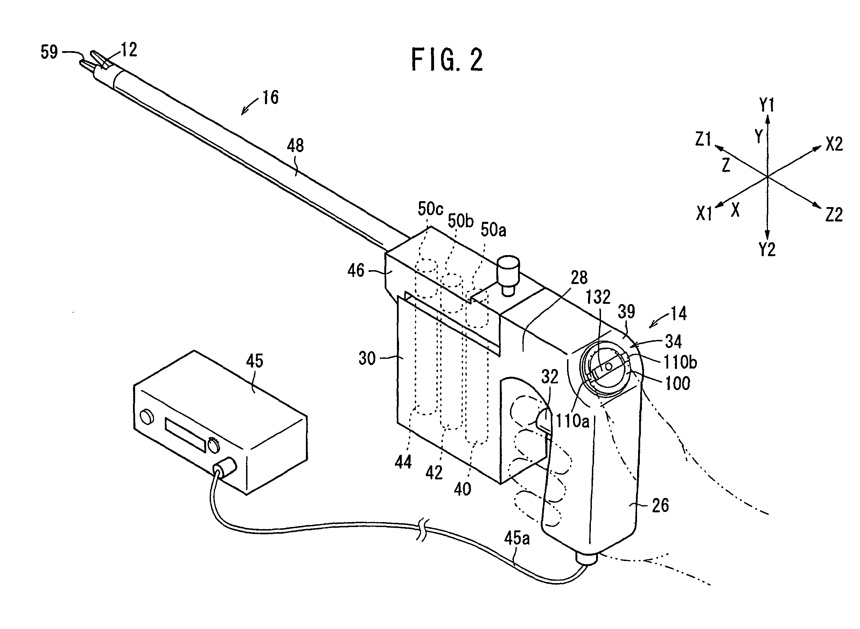

As shown in FIGS. 1 and 2, the manipulator 10 comprises an operation command unit (operating unit) 14 on a proximal end thereof, which is held and operated by hand, a working unit 12 on a distal end thereof for working on a living tissue, and an elongate connector 16 interconnecting the working unit 12 and the operation command unit 14. The working unit 12 and the connector 16 have small diameters and can be inserted into a body cavity 22 through a trocar 20, in the form of a hollow cylinder mounted in an abdominal region or the like of the patient. The working unit 12 is actuated by the operation command unit 14 in order to perform various techniques to grip, remove, and/or to perform suture knot tying on an affected part of the patient's body inside of the body cavity 22.

In the following description, it shall be assumed that transverse directions of the manipulator 10 are designated as X directions, vertical directions as Y directions, and longitudinal directions of the connector 16 as Z directions. With respect to the X directions, the rightward direction is designated as an X1 direction, and the leftward direction is designated as an X2 direction. With respect to the Y directions, the upward direction is designated as a Y1 direction, and the downward direction is designated as a Y2 direction. With respect to the Z directions, the forward direction is designated as a Z1 direction, and the rearward direction is designated as a Z2 direction. Unless otherwise noted, these directions represent directions of the manipulator 10 when it is in a neutral posture. Definitions of the above directions are given for illustrative purposes only, and the manipulator 10 can be used in any orientation, e.g., it may be used upside down.

The operation command unit 14 includes a grip handle 26, which is gripped by the hand, a bridge 28 extending from an upper portion of the grip handle 26, and an actuator block 30 connected to a distal end of the bridge 28. The grip handle 26 has a lower end connected to a lower end of the actuator block 30.

The grip handle 26 extends in the Y2 direction from the end of the bridge 28, and is of a length that is suitable for being gripped by hand. The grip handle 26 includes a trigger lever 32 and a composite input unit 34, which serve as an input means. The trigger lever 32 is positioned slightly beneath the bridge 28, projecting slightly in the Z1 direction. The trigger lever 32 is disposed in a position where it can easily be pulled by the index finger of the hand while gripping the grip handle 26.

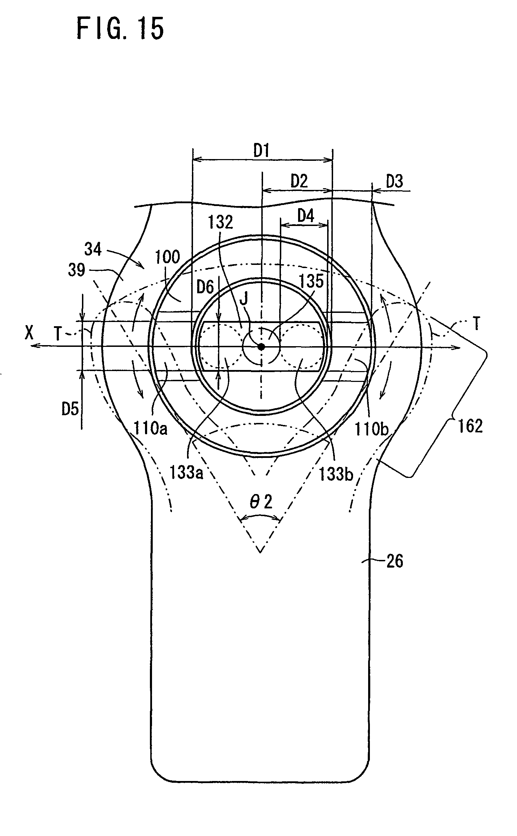

The composite input unit 34 serves as a composite input means for applying rotational commands to the working unit 12 in both rolling directions (directions about the axis) and in yawing directions (leftward and rightward directions). The composite input unit 34 has a circular shape when viewed in front elevation (see FIG. 15), and is disposed on a flat area 39 of the joint between the upper end of the grip handle 26 and the bridge 28. As can be seen from FIG. 2, the composite input unit 34 is disposed in a position where it can easily be operated by the thumb of the hand, while gripping the grip handle 26.

The flat area 39 has a substantially annular shape, which is larger in diameter than the composite input unit 34. When the composite input unit 34 is not operated, the user, typically a surgeon, places the thumb on the flat area 39, so that the user can firmly grip the grip handle 26 without touching the composite input unit 34. A line normal to the flat area 39 and the surface of the composite input unit 34 extends along a direction that lies substantially at an intermediate position between the Z2 direction and the Y1 direction. Therefore, the user can have the finger pad T of the thumb held naturally against the flat area 39 and the surface of the composite input unit 34. The finger pad T refers to the portion of the thumb, which extends from the first joint (closest to the fingertip) to the fingertip, and which lies on the same side as the palm of the hand. Details of the composite input unit 34 shall be described later.

The actuator block 30 houses therein three motors 40, 42, 44, corresponding to respective mechanisms providing three degrees of freedom, which are incorporated in the working unit 12. The motors 40, 42, 44 are arrayed in parallel with each other in the longitudinal direction of the connector 16. The motors 40, 42, 44 are small in size and diameter, thereby allowing the actuator block 30 to have a compact flat shape. The actuator block 30 is disposed downwardly from the end of the operation command unit 14 in the Z1 direction. The motors 40, 42, 44 are energized in order to rotate their drive shafts, under the control of a controller 45, based on operations of the operation command unit 14.

The controller 45, which serves to electrically control the manipulator 10, is connected by a cable 45a to a connector on the lower end of the grip handle 26.

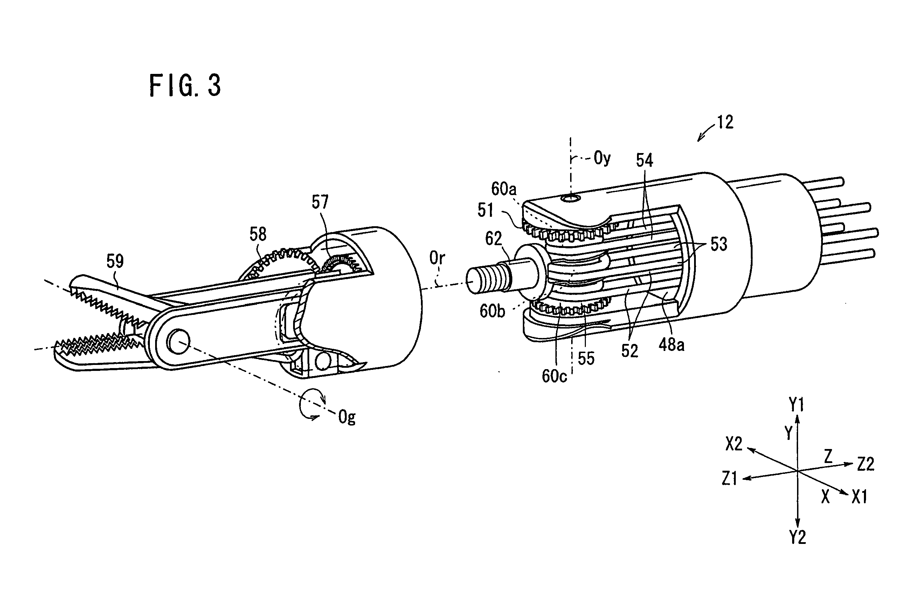

The connector 16 includes a joint 46 joined to the actuator block 30, and a hollow connector shaft 48 that extends in the Z1 direction from the joint 46. The joint 46 houses therein drive pulleys 50a, 50b, 50c, which are connected respectively to the drive shafts of the motors 40, 42, 44. Wires 52, 53, 54 (see FIG. 3) are trained respectively around the pulleys 50a, 50b, 50c, wherein the wires 52, 53, 54 extend through a space 48a in the connector shaft 48 to the working unit 12. The wires 52, 53, 54 may be of the same type and diameter.

The joint 46 can be manually operated according to a predetermined process in order to disconnect the connector 16 from the operation command unit 14, to perform cleaning, sterilization, maintenance, etc. The connector 16 and the working unit 12 can be replaced with other connectors and working units. For example, depending on the technique required for a certain surgical operation, the connector 16 may be replaced by a connector having a different length and/or the working unit 12 may be replaced by a working unit incorporating different mechanisms.

As shown in FIG. 3, the working unit 12 incorporates therein mechanisms possessing three degrees of freedom. These mechanisms include a mechanism (tilting mechanism, pivot shaft) having a first degree of freedom for angularly moving a portion of the working unit 12, which is positioned ahead of a first rotational axis Oy extending along the Y directions, in yawing directions about the first rotational axis Oy, a mechanism (rolling mechanism) having a second degree of freedom for angularly moving the portion of the working unit 12 in rolling directions about a second rotational axis Or extending along the Z directions, and a mechanism for opening and closing a gripper (opening and closing mechanism) 59 about a third rotational axis Og extending along the X directions.

The first rotational axis Oy of the yawing mechanism, having the first degree of freedom, extends non-parallel to an axis C of the connector 16, which extends from the proximal end to the distal end of the connector 16. The second rotational axis Or of the rolling mechanism, having the second degree of freedom, extends along an axis of the working unit 12 at the tip end thereof, i.e., the gripper 59, so that the gripper 59 can roll around the second rotational axis Or.

The working unit 12 is actuated by the wires 54, 53, 52, which are trained around respective tubes 60a, 60b, 60c inside of the working unit 12.

In the working unit 12, when the wires 52, 54 and 53 are actuated, a gear 55, a gear 51 and a main shaft 62 are rotated respectively. When the gears 51, 55 and the main shaft 62 are rotated at the same angle, the working unit 12 is rotated in the yawing direction. When the gear 55 is rotated, a face gear, not shown, is rotated. When the gear 51 is rotated, a face gear 57 is rotated. When the unillustrated face gear and the face gear 57 are rotated at the same angle, the working unit is rotated in the rolling direction. When the unillustrated face gear and the face gear 57 are rotated at different angles, the gear 58 is relatively rotated so as to open and close the gripper 59.

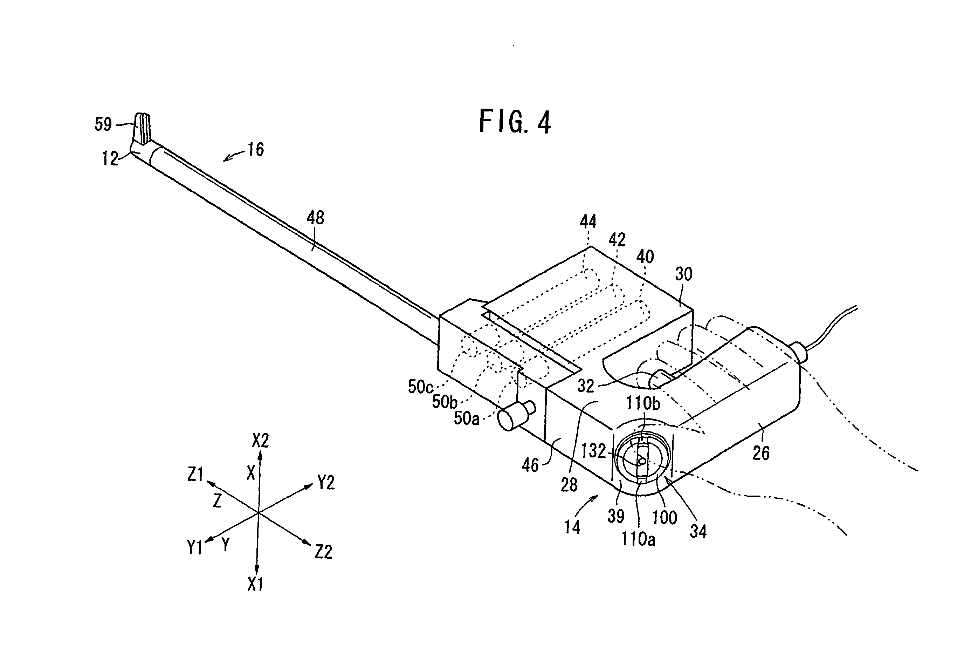

The working unit 12 does not incorporate a mechanism having a degree of freedom for moving the working unit 12 in pitching directions, i.e., vertical directions. However, when the grip handle 26 is oriented horizontally, so as to tilt the manipulator 10 as a whole through 90° as shown in FIG. 4, movement of the working unit 12 in the yawing direction is converted into movement in the pitching direction. Consequently, the lack of such a pitching mechanism does not pose any practical problems.

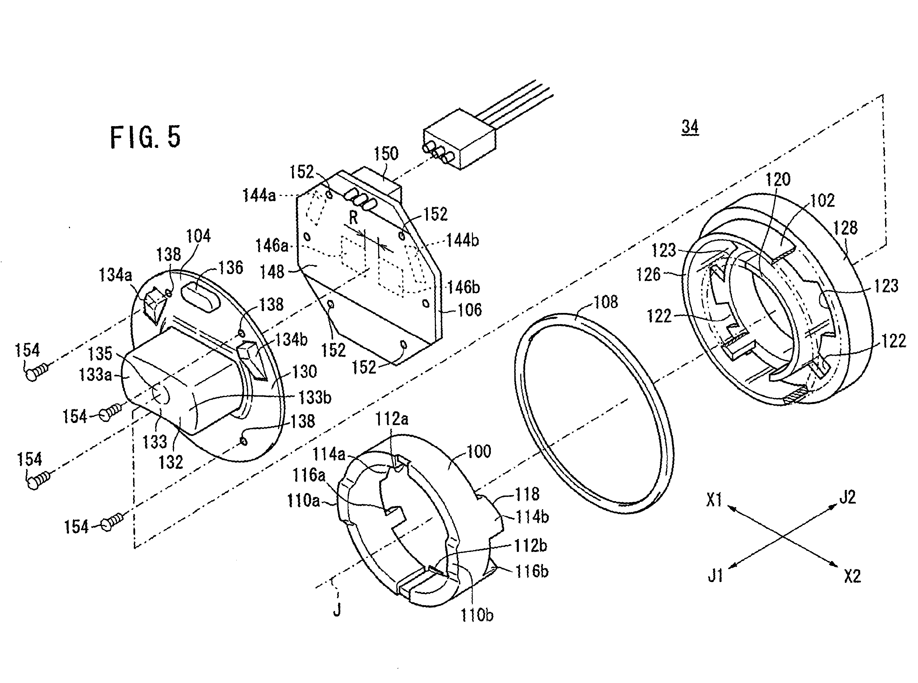

As shown in FIG. 5, the composite input unit 34 includes a shuttle ring (first input member) 100, a substantially hollow cylindrical base body 102, a rubber pad 104, a board 106, and an 0-ring 108. It is assumed that the composite input unit 34 has a central axis J, wherein the direction along the central axis J oriented toward the viewer of FIG. 5 is designated as a J1 direction, and the direction along the central axis J oriented away from the viewer of FIG. 5 is designated as a J2 direction. The shuttle ring 100 is inserted into an inner cavity of the base body 102 along the J1 direction, and the rubber pad 104 is inserted into the inner cavity of the base body 102 along the J2 direction.

The shuttle ring 100 serves as an input means for applying a rolling command to the working unit 12. As the operating amount applied to the shuttle ring 100 increases, the rotational speed of the working unit 12 is set to become faster. Further, when the shuttle ring 100 is not operated, the working unit 12 is held at rest in the rolling directions.

The shuttle ring 100 includes a pair of knobs (finger holders) 110a, 110b disposed in diametrically symmetric positions on the side face of the shuttle ring 100 facing in the J1 direction, a pair of engaging teeth 112a, 112b disposed in upper and lower positions on the inner circumferential surface of the shuttle ring 100, a pair of protrusions 114a, 114b disposed in upper symmetric positions on the side face of the shuttle ring 100 facing in the J2 direction, and a pair of thin stoppers 116a, 116b disposed in lower symmetric positions on the side face of the shuttle ring 100 that faces in the J2 direction.

The shuttle ring 100 has an inside diameter D1 (distance between the knobs 110a, 110b, see FIG. 15) of 25 mm. Therefore, the knob 110a or 110b is spaced from the center of rotation of the shuttle ring 100 by a distance D2 (see FIG. 15) of 12.5 mm. The inside diameter D1 should preferably be large enough to allow a pad 132, to be described later, to be placed in the shuttle ring 100, and should preferably be kept within a range of thumb movement so that the pad 132 can easily be operated by the thumb. In view of these considerations, the inside diameter D1 should be within the range of from 20 mm to 40 mm (the distance D2 falls within the range of from 10 mm to 20 mm), and more preferably, should be within the range of from 25 mm to 30 mm (the distance D2 falls within the range of from 12.5 mm to 15 mm).

The shuttle ring 100 has a width D3 (see FIG. 15) of 5 mm, which is suitable for the thumb to be neatly applied to the shuttle ring 100, and also has an outside diameter of 35 mm (= D1 + D3 × 2 = 25 + 5 × 2). The width D3 should be within the range of from 5 mm to 10 mm, which is suitable for the thumb to be neatly applied to the shuttle ring 100.

The knobs 110a, 110b have a shape slightly protruding in the J1 direction, so that the finger pad T of the thumb can effectively be applied to the knobs 110a, 110b. For example, each of the knobs 110a, 110b should have a height within the range of from 1 mm to 5 mm, and a circumferential length D5 (see FIG. 15) within the range of from 3 mm to 10 mm. The knobs 110a, 110b are disposed in symmetrical positions, which are spaced from each other diametrically across the axis J.

The protrusions 114a, 114b have respective slanted surfaces 118.

The base body 102 has an inner tube 120, disposed in the inner cavity thereof, and an outer tube 126, disposed radially outwardly of the inner tube 120. The inner cavity of the base body 102 includes two guide holes 122, two holes 123 in which the protrusions 114a, 114b are inserted, and engaging portions (not shown), wherein the engaging teeth 112a, 112b engage with the engaging portions and undergo circumferential movement therewith. The distance between the inner tube 120 and the outer tube 126 is substantially equal to the width D3, so that the shuttle ring 100 can be angularly movable between the inner tube 120 and the outer tube 126. The stoppers 116a, 116b are inserted respectively into the guide holes 122. The shuttle ring 110 is angularly movable within a range defined by the ends of the guide holes 122, which are engageable by the stoppers 116a, 116b.

Specifically, the shuttle ring 110 is angularly movable within a range of ± 10° about its central position. The angularly movable range of the shuttle ring 110 should be large enough to allow the user to move the shuttle ring 110 effectively, e.g., to move the shuttle ring 110 a small interval, and the range should preferably be within a movable range of the finger pad T, so as to allow the user to move the shuttle ring 110 easily with the finger pad T. In view of such considerations, the angularly movable range of the shuttle ring 110 should preferably be within a range of from ± 5° to ± 20° and, more preferably, should be within a range of from ± 5° to ± 10°.

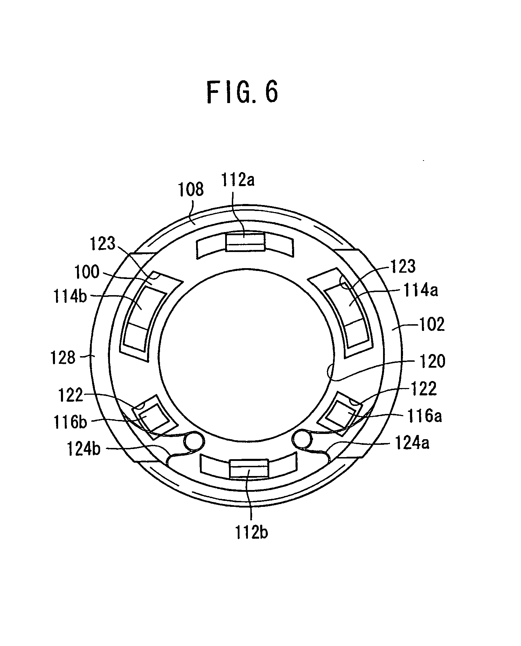

As shown in FIG. 6, the base body 102 has two springs 124a, 124b on a surface thereof facing in the J2 direction. When the shuttle ring 100 is turned clockwise, as viewed in front elevation (i.e., counterclockwise in FIG. 6), the stopper 116a compresses the spring 124a, which produces a force that acts to return the shuttle ring 100 counterclockwise. Conversely, when the shuttle ring 100 is turned counterclockwise, as viewed in front elevation (i.e., clockwise in FIG. 6), the stopper 116b compresses the spring 124b, which produces a force that acts to return the shuttle ring 100 clockwise. In this manner, when the shuttle ring 100 is not operated, the shuttle ring 100 is pushed back by the springs 124a, 124b, i.e., the resilient forces of the springs 124a, 124b are held in equilibrium, such that the shuttle ring 100 is maintained in a neutral position, as shown in FIG. 15.

The base body 102 has a flange 128 on an end thereof facing in the J2 direction. Axial ends of the inner and outer tubes 120, 126 are joined to the flange 128. The O-ring 108 is held against the flange 128.

As shown in FIGS. 5, 7 and 8, the rubber pad 104 comprises a thin circular sheet 130, a pad (second input member) 132 projecting from the circular sheet 130 in the J1 direction, a pair of bearing ledges 134a, 134b disposed symmetrically on the circular sheet 130 and projecting in the J1 direction, and a clearance 136 defined in an upper portion of the circular sheet 130. The circular sheet 130 has a plurality of screw holes 138 defined therein.

The pad 132 serves as an input means according to the invention for applying a yawing command to the working unit 12. As the operating amount applied to the pad 132 increases, the speed at which the working unit 12 is tilted in a yawing direction increases. When the pad 132 is not operated, the working unit 12 is held at rest in the yawing directions.

The pad 132 is in the form of a protrusion, having upper and lower surfaces extending parallel to each other, as well as arcuate left and right sides. The arcuate left and right ends have a radius of curvature corresponding to the radius of curvature of inner circumferential surfaces of the inner tube 120 and the shuttle ring 100 (see FIG. 15).

The pad 132 has an end 133 facing in the J1 direction and having a curved surface, including a slightly dented central area and left and right slanted surfaces 133a, 133b, extending from the central area toward the left and right sides thereof. The end 133 has a low boss 135 in the central area thereof, which allows the left and right slanted surfaces 133a, 133b to be easily recognizable by tactile sensation. Each of the left and right slanted surfaces 133a, 133b has a width D4 of 5 mm, which is suitable for the thumb to be neatly applied thereto. As with the width D3, the width D4 (see FIG. 15) should be within a range of from 5 mm to 10 mm.

The pad 132 has a width D6 of 17 mm in a direction perpendicular to the direction in which the pad 132 is tiltable. The width D6 is suitable for natural positioning of the thumb and corresponds to the movable range of the thumb. The width D6 should be within a range of from 10 mm to 25 mm, for allowing the thumb to operate the pad 132 easily.

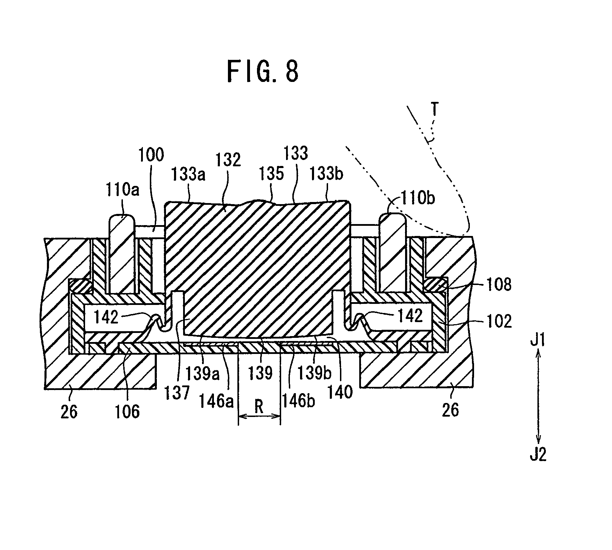

As shown in FIG. 8, the pad 132 comprises a solid member, making up a substantially half portion of the pad 132 which extends in the J1 direction, and a protrusion 137, making up a remaining substantially half portion thereof which extends in the J2 direction. The pad 132 further includes an outer wall, surrounding the protrusion 137 and spaced from the protrusion 137 by a gap. The protrusion 137 has an end surface 139 facing in the J2 direction, and which is spaced slightly from the circular sheet 130 in the J1 direction. The end surface 139 and the board 106 are spaced from each other by a gap 140.

The gap 140 (along with gaps 145, 160, 187 and 188 to be described later) is set to about 1 mm, for example.

The pad 132 and the circular sheet 130 are joined to each other by a joint, including a folded web 142. When the end face 133 is pushed by the finger, the pad 132 can easily be tilted in a given direction depending on whether the left slanted surface 133a or the right slanted surface 133b is pressed. When the pad 132 is not pushed, the pad 132 automatically springs back to its neutral position due to the resilient force of the folded web 142.

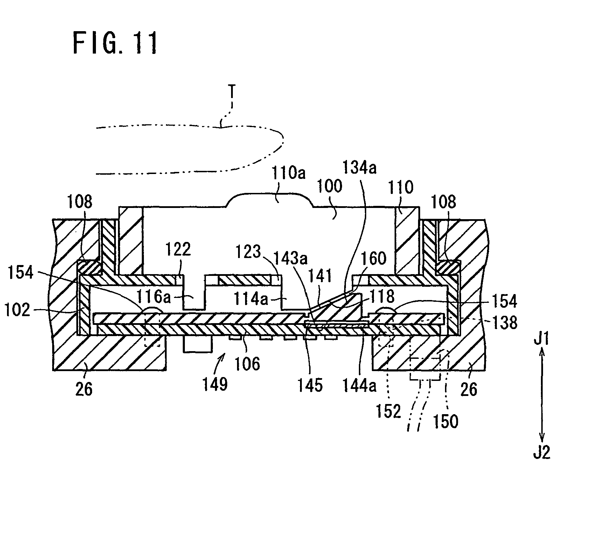

Each of the bearing ledges 134a, 134b has a slanted surface 141 (see FIG. 11), having an upper end which is higher than the lower end thereof. The bearing ledges 134a, 134b are disposed slightly upwardly from the respective protrusions 114a, 114b. When the pad 132 is in a neutral position, the slanted surfaces 118 and the slanted surfaces 141 face each other.

The circular sheet 130 has shallow recesses 143a, 143b defined in a surface thereof behind the respective bearing ledges 134a, 134b, and providing gaps 145 between the bearing ledges 134a, 134b and the board 106. In order to secure the gaps 145, small protrusions 147 are provided on the recesses 143a, 143b, the protrusions 147 being held in point contact with the board 106.

The board 106 has a hexagonal shape. The board 106 supports, on a surface thereof facing in the J1 direction, a turn-detecting first pressure-sensitive sensor 144a, a turn-detecting second pressure-sensitive sensor 144b, a tilt-detecting first pressure-sensitive sensor 146a, and a tilt-detecting second pressure-sensitive sensor 146b. The sensors are formed by fine printed patterns, which are covered with a thin resin sheet 148 such that the sensors are not directly exposed to air.

The sensors 144a, 144b, 146a, 146b may be of any of various types, which are capable of detecting angular displacements of the shuttle ring 100 and tilting of the pad 132. For example, the sensors 144a, 144b, 146a, 146b may comprise resistive sensors made up of conductive plastics, semiconductor sensors, optical sensors, or the like. The sensors 144a, 144b, 146a, 146b need not necessarily be able to continuously detect angular displacements and tilts, but may be combined with an on-off switch that detects only a neutral position or a direction of the angular displacements and tilts.

The turn-detecting first pressure-sensitive sensor 144a and the turn-detecting second pressure-sensitive sensor 144b are disposed in respective positions, i.e., in the recesses 143a, 143b, facing the surface of the circular sheet 130 behind the bearing ledges 134a, 134b. The tilt-detecting first pressure-sensitive sensor 146a and the tilt-detecting second pressure-sensitive sensor 146b are disposed in respective positions, facing respective left and right portions 139a, 139b of the end surface 139 of the pad 132.

A plurality of electronic parts 149 (see FIG. 11), for adjusting detected signals from the sensors 144a, 144b, 146a, 146b and supplying such adjusted signals to the controller 45, are mounted on the surface of the board 106 that faces in the J2 direction. The board 106 comprises a connector 150 connected to the controller 45, and a plurality of screw holes 152 through which the board 106 is fastened to the grip handle 26 by screws. The connector 150 has terminal ends slightly projecting in the J1 direction and accommodated within the clearance 136 for protecting the circular sheet 130 from damage. The screw holes 138 and the screw holes 152 are held in registry with each other. Screws 154 are inserted into the screw holes 138 and 152 in order to firmly secure the circular sheet 130 and the board 106 to each other (see FIG. 11).

As shown in FIG. 8, when the thumb is spaced from the pad 132, the pad 132 is held in a neutral position under the resilient force of the folded web 142.

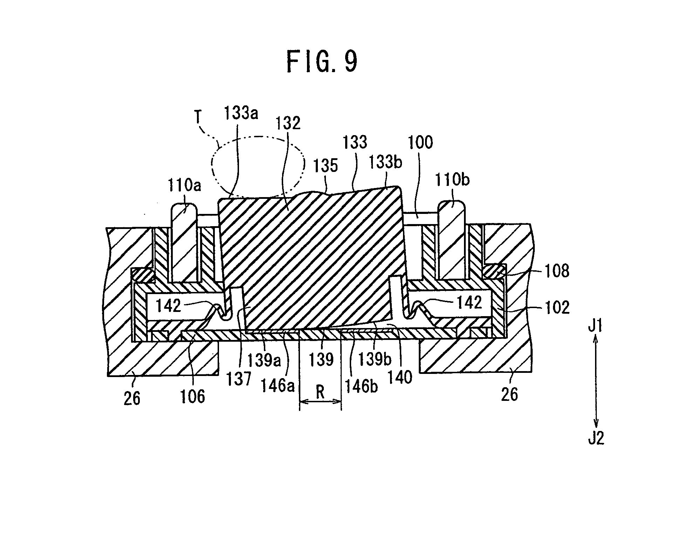

As shown in FIG. 9, when the finger pad T of the thumb is applied to the left slanted surface 133a of the pad 132 and pushed down to tilt the pad 132 to the left, the left portion 139a of the end surface 139 of the protrusion 137 is pressed against the board 106, thereby pressing the tilt-detecting first pressure-sensitive sensor 146a. Because the protrusion 137 presses the tilt-detecting first pressure-sensitive sensor 146a more strongly when the left portion 133a is pushed down more deeply, tilting of the pad 132 to the left and/or the pressing force of the pad 132 can be detected as a result of signals generated by the tilt-detecting first pressure-sensitive sensor 146a.

When the right slanted surface 133b of the pad 132 is pushed down to tilt the pad 132 to the right, the right portion 139b of the end surface 139 presses against the tilt-detecting second pressure-sensitive sensor 146b. Tilting of the pad 132 to the right and/or the pressing force of the pad 132 can be detected as a result of signals generated by the tilt-detecting second pressure-sensitive sensor 146b.

As seen in FIG. 8, the tilt-detecting first pressure-sensitive sensor 146a and the tilt-detecting second pressure-sensitive sensor 146b are spaced from each other by a suitable distance R such that, when the pad 132 is in its neutral position, a gap 140 exists between the end surface 139 and the board 106.

Therefore, even if the pad 132 is slightly tilted, no pressing force is applied on the tilt-detecting first pressure-sensitive sensor 146a and the tilt-detecting second pressure-sensitive sensor 146b, so that the sensors keep outputting signals indicative of the neutral position. As a consequence, a certain dead zone exists before the tilt-detecting first pressure-sensitive sensor 146a and the tilt-detecting second pressure-sensitive sensor 146b detect that the pad 132 has been tilted. Even when the user inadvertently presses the central boss 135 on the end 133, since the tilt-detecting first pressure-sensitive sensor 146a and the tilt-detecting second pressure-sensitive sensor 146b are spaced from each other, the sensors are not touched by the end surface 139 and continue to output signals indicative of the neutral position.

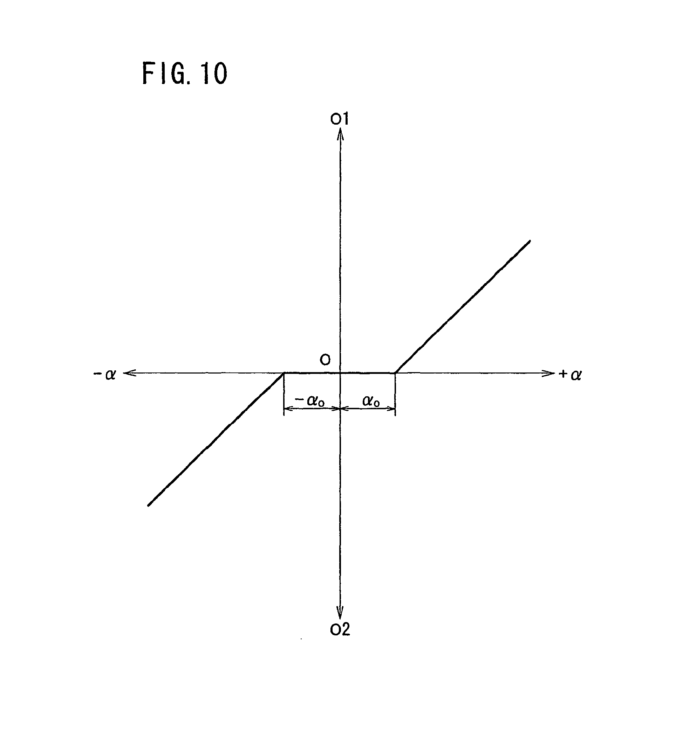

Specifically, as shown in FIG. 10, a pressing force that presses the left portion 133a of the pad 132 is represented by -α, a pressing force that presses the right portion 133b is represented by +α, an output 01 of the tilt-detecting first pressure-sensitive sensor 146a is indicated in the plus direction of the longitudinal axis, and an output 02 of the tilt-detecting second pressure-sensitive sensor 146b is indicated in the minus direction of the longitudinal axis. In this case, both the outputs 01 and 02 are zero in the range of ± α₀ with reference to the original point as the neutral position, and thus a dead zone is obtained. The dead zone serves as play in the movement of the pad 132, whereby response sensitivity of the operating unit is suitably reduced and a user can easily operate the pad 132. The distance R may be within a range of from 2 mm to 8 mm in order to provide a suitable dead zone.

Due to the dead zone existing within the tilting movement, when the user holds the thumb out of contact with the pad 132, the tilt-detecting first pressure-sensitive sensor 146a and the tilt-detecting second pressure-sensitive sensor 146b reliably output signals indicating the neutral position, thereby preventing the working unit 12 from being tilted in a yawing direction against the will of the user.

Further, the tilt-detecting first pressure-sensitive sensor 146a and the tilt-detecting second pressure-sensitive sensor 146b are suitably spaced away from each other, and the end surface 139 is gently convex downward. Owing to this structure, even if the pad 132 is pushed down without the tilting motion, no pressure is applied to the tilt-detecting first pressure-sensitive sensor 146a or the tilt-detecting second pressure-sensitive sensor 146b. Accordingly, the working unit 12 is not operated in the yawing direction unintentionally.

The above-mentioned pushing operation of the pad 132 may be regarded as a preparing operation. Further, the user may recognize that the working unit 12 is operated in the yawing direction by tilting the pad 132 after the preparing operation. In this case, since the preparing operation, i.e., the pushing operation of the pad 132 is added before the tilting operation of the pad 132, the operation for moving the working unit 12 in the yawing direction is regarded as a two-stage operation. Such a two-stage operation is effective in operating the working unit 12 slowly and reliably.

Further, even if the pad 132 is tilted within a small angular range or if the central boss 135 is pushed down, the working unit 12 is not moved angularly in the yawing directions. Such play in the movement of the pad 132 allows the user to easily operate the pad 132. The distance R should be within a range of from 2 mm to 8 mm in order to provide a moderate dead zone.

As shown in FIG. 11, when the thumb is spaced from the shuttle ring 100, the shuttle ring 100 is held in a neutral position under the resiliency of the springs 124a and 124b. When the protrusions 114a, 114b engage with the bearing ledges 134a, 134b, due to resiliency of the rubber pad 104, the protrusions 114a, 114b are subjected to forces tending to return the protrusions 114a, 114b. Therefore, the shuttle ring 100 also is held in a neutral position by the bearing ledges 134a, 134b.

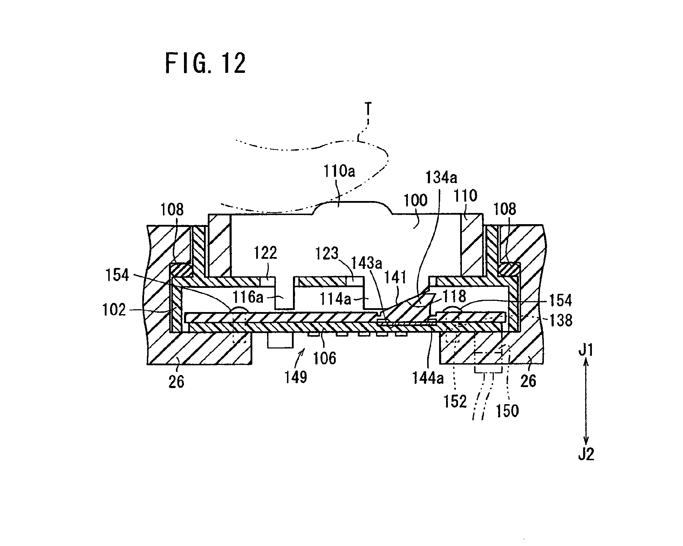

As shown in FIG. 12, when the finger pad T of the thumb is applied to the knob 110a and pushes the knob 110a to turn the shuttle ring 100 clockwise, the slanted surface 118 of the protrusion 114a rides over the slanted surface 141 of the corresponding bearing ledge 134a, thereby deforming the bearing ledge 134a to bring the reverse surface thereof into contact with the board 106 and pressing the turn-detecting first pressure-sensitive sensor 144a. Since the bearing ledge 134a presses against the turn-detecting first pressure-sensitive sensor 144a more forcefully as the protrusion 114a is pushed more, the angular displacement of the shuttle ring 100 in the clockwise direction can be detected as a result of the signal produced by the turn-detecting first pressure-sensitive sensor 144a.

When the thumb is applied to the knob 110b and pulls the knob 110b so as to turn the shuttle ring 100 clockwise, the shuttle ring 100 also is moved angularly clockwise. Therefore, the angular displacement of the shuttle ring 100 in the clockwise direction can be detected as a result of the signal produced by the turn-detecting first pressure-sensitive sensor 144a.

When the thumb is applied to the knob 110b and pushes the knob 110b, or the thumb is applied to the knob 110a and pulls the knob 110a, so to move the shuttle ring 100 angularly counterclockwise, the protrusion 114b presses down on the bearing ledge 134b, thereby pressing the turn-detecting second pressure-sensitive sensor 144b. Therefore, angular displacement of the shuttle ring 100 in the counterclockwise direction can be detected as a result of the signal produced by the turn-detecting second pressure-sensitive sensor 144b.

As can be seen from FIG. 11, when the shuttle ring 100 is in the neutral position, a small gap 160 exists between the slanted surface 118 and the slanted surface 141. In addition, a small gap 145 exists between the circular sheet 130 and the board 106. Therefore, even if the shuttle ring 100 is turned slightly, the shuttle ring 100 does not apply a pressing force to the turn-detecting first pressure-sensitive sensor 144a or to the turn-detecting second pressure-sensitive sensor 144b, and the sensors continue to output signals indicative of the neutral position. As a consequence, a certain dead zone exists before the turn-detecting first pressure-sensitive sensor 144a and the turn-detecting second pressure-sensitive sensor 144b detect that the shuttle ring 100 has been turned. Stated otherwise, like FIG. 10, outputs of the turn-detecting first pressure-sensitive sensor 144a and the turn-detecting second pressure-sensitive sensor 144b are zero within a predetermined range taking the center as a reference position, thereby obtaining a dead zone.

Due to the angular movement dead zone, when the user takes his thumb out of contact with the shuttle ring 100, the turn-detecting first pressure-sensitive sensor 144a and the turn-detecting second pressure-sensitive sensor 144b reliably output signals indicative of the neutral position. Accordingly, the working unit 12 is prevented from being turned in a rolling direction against the will of the user. Due to the angular movement dead zone, when the user takes his thumb out of contact with the shuttle ring 100, even if the shuttle ring 100 is slightly displaced from a neutral state (ex. two springs 124a and 124b do not balance with each other), the turn-detecting first pressure-sensitive sensor 144a and the turn-detecting second pressure-sensitive sensor 144b reliably output signals indicative of the neutral position. Accordingly, the working unit 12 is prevented from being turned in a rolling direction uselessly.

Even when the shuttle ring 100 is turned inside of a small angular range, the working unit 12 is not moved angularly in the rolling directions. Such play in movement of the shuttle ring 100 enables easy operation of the manipulator 10.

Such a dead zone preferably should be within a range of from ± 1° to ± 8° in terms of angular displacements of the shuttle ring 100.

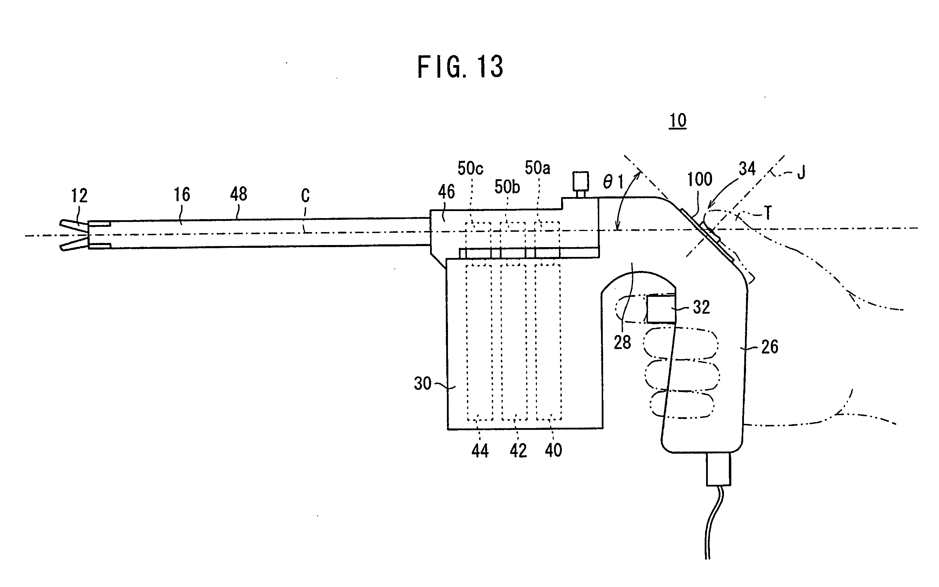

Such a dead zone as indicated by ± α₀ in FIG. 10 can be obtained by a software process in the controller 45. Further, a more reliable dead zone can be obtained by a mechanical structure based on the presence of gaps 145, 160 and the distance R. For example, if there is no gap between the tilt-detecting first pressure-sensitive sensor 146a and the tilt-detecting second pressure-sensitive sensor 146b, and the pad 132 is pushed down, both the tilt-detecting first pressure-sensitive sensor 146a and the tilt-detecting second pressure-sensitive sensor 146b output signals that are based on the pressing force. In this case, it is difficult to form an appropriate dead zone by a software process. Such a difficulty causes complicated procedures, an increased program size and a longer debugging time. Compared with this, since the manipulator 10 provides a dead zone by the above-mentioned mechanical structure, the above inconveniences are not caused. As shown in FIG. 13, the angle θ1 formed between the axis C of the connector shaft 48 and the plane in which the shuttle ring 100 rotates, i.e., the angle formed between surfaces of the composite input unit 34 and the flat area 39, is set to 45°. The angle θ1 preferably should be within a range of from 35° to 55°, so that the shuttle ring 100 matches the natural positioning of the thumb as well as the movable range of the thumb.

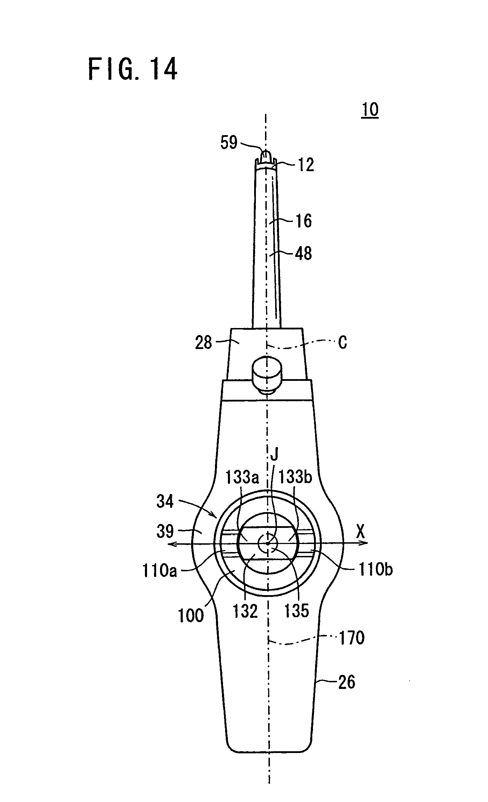

As shown in FIG. 14, the shuttle ring 100 has a vertical central line 170, which is aligned with the longitudinal axis C of the connector shaft 48 when viewed along the rotational axis J of the shuttle ring 100. Consequently, the user feels as though the rolling mechanism of the working unit 12 operates concentrically with the shuttle ring 100, and in direct relationship thereto. Therefore, the user can easily operate the manipulator 10.

Furthermore, since it is horizontally symmetrical in shape, the manipulator 10 can be used by either the right hand or the left hand. In FIG. 14, the manipulator 10 is illustrated in perspective, for enabling understanding of the way in which the manipulator 10 is seen from the viewpoint of the user.

As shown in FIG. 15, in the composite input unit 34, the pad 132, which is centered about the J axis, and the shuttle ring 100 are disposed coaxially in a concentrated and compact configuration.

When the user applies the thumb to either one of the knobs 110a, 110b and pushes the knobs upwardly so as to turn the shuttle ring 100 in one direction, the working unit 12 is turned (i.e., rolls) in the same direction. Therefore, the rolling mechanism of the working unit 12 and the shuttle ring 100 both turn in the same direction, so that the user is able to operate the working unit 12 easily and intuitively. Furthermore, the single shuttle ring 100 allows the rolling mechanism to turn in opposite directions. Therefore, the shuttle ring 100 does not require an increased number of input members, but is simple in structure and easy to operate.

Since the shuttle ring 100 has an annular shape, the shuttle ring 100 is easily visually recognized as the input means for entering rolling commands. Further, the user can easily learn and will not quickly forget how to use the shuttle ring 100. However, the operation command unit 14 may also be designed to incorporate another input means for entering rolling commands. For example, the operation command unit 14 may comprise an input means which turns in an arc and can be moved angularly around the axis J, wherein only the knobs 110a, 110b are exposed on the surface, or a C-shaped ring that is partially open.

Although the shuttle ring 100 and the pad 132 are disposed in a concentrated fashion, since they are mechanically separate from each other, the user can easily operate the shuttle ring 100 and the pad 132 separately from each other, and the user does not become confused when using them.

The pad 132 is located inside of the shuttle ring 100, and hence the pad 132 is compact and can be operated by moving the thumb within a small range. The single pad 132 allows the yawing mechanism to turn in opposite yawing directions. Therefore, the pad 132 does not require an increased number of input members, but is simple in structure and easy to operate.

The knobs 110a, 110b and the pad 132 are juxtaposed in the X direction, and disposed on and inside of the shuttle ring 100, which has an appropriate diameter (D1 + D3 × 2 = 35 mm). The knobs 110a, 110b and the pad 132 are thus positioned inside of the movable range of the finger pad T of the thumb, and thus can be operated highly effectively.

Specifically, when the user grips the grip handle 26, the finger pad T of the thumb is naturally placed near to the central boss 135 of the pad 132. If the thumb is movable within a general range defined by an angle θ2, which is achieved when the second joint (central joint) of the thumb and the third joint (proximal joint) of the thumb are moved, then the knobs 110a, 110b and the pad 132 are positioned inside of an arcuate range 162, in which the finger pad T moves. Accordingly, the knobs 110a, 110b and the pad 132 can be operated in a concentrated fashion by the thumb, without causing any undue motion of the thumb.

The angular movable range of the shuttle ring 100 has an appropriately wide range of ±10° regulated by the stoppers 116a, 116b. Therefore, the user can move the shuttle ring 110 by a small interval, and can move the shuttle ring 110 easily with the finger pad T.

The surface of the composite input unit 34 lies substantially flush with the flat area 39, except that the knobs 110a, 110b and the boss 135 project slightly from the surface of the composite input unit 34. Therefore, when the user moves the finger pad T in X directions, the finger pad T is simply moved along the substantially flat surface of the composite input unit 34, whereby the user can operate the composite input unit 34 easily.

For performing surgical operation techniques using the manipulator 10, the user operates the composite input unit 34 as follows:

First, the user applies the finger pad T of the thumb lightly to the boss 135. When the finger pad T of the thumb rests lightly on the boss 135, the thumb is referred to as being in a basic posture.

In order to move the working unit 12 to the left in a yawing direction, the user moves the finger pad T from the boss 135 onto the left slanted surface 133a and depresses the left slanted surface 133a. Since the left slanted surface 133a is free of the boss 135, and also is positioned to the right of the knob 110a, the user can easily confirm the position of the left slanted surface 133a through tactile sensation, without requiring the user to look at the hand. In order to move the working unit 12 to the right in a yawing direction, the user can depress the right slanted surface 133b, and also can easily confirm the position of the right slanted surface 133b, in the same manner as the position of the left slanted surface 133a.

When the working unit 12 is moved to any one of the right or the left in the yawing direction, tilts in a minute range with reference to the neutral position of the pad 132 are not detected by the tilt-detecting first pressure-sensitive sensor 146a or the tilt-detecting second pressure-sensitive sensor 146b, owing to the presence of a dead zone. Accordingly, the working unit 12 is not moved in the yawing direction. Thus, sensitivity of the manipulator does not become too high in operation by the user, thereby enabling an easy operation.

For rolling the working unit 12, the user moves the finger pad T further to the left until the finger pad T hits against the knob 110a, and then pushes the knob 110a upwardly or pulls the knob 110a downwardly. When the user moves the finger pad T to the left along the surface of the composite input unit 34, the finger pad T naturally touches the knob 110a. Therefore, the user can easily confirm the position of the knob 110a. Alternatively, the user can operate the knob 110b to roll the working unit 12, wherein the user can easily confirm the position of the knob 110b, similar to the position of the knob 110a. Since the knobs 110a, 110b protrude appropriately from the surface of the composite input unit 34, the knobs 110a, 110b can easily be pushed upwardly or pulled downwardly with a light force.

When the working unit 12 is turned either clockwise or counterclockwise, angular displacements in a minute range with reference to the neutral position of the shuttle ring 100 are not detected by the turn-detecting first pressure-sensitive sensor 144a or the turn-detecting second pressure-sensitive sensor 144b, owing to the presence of a dead zone. Accordingly, the working unit 12 is not turned in the rolling direction. Thus, sensitivity of the manipulator does not become too high in operation by the user, thereby enabling an easy operation.

When the user does not wish to operate the composite input unit 34, the user places the thumb on the flat area 39. If the user wants to move the working unit 12 in a rolling or yawing direction with the thumb, after the thumb has been placed on the left end of the flat area 39, then the user moves the finger pad T to the right along the flat area 39 and the composite input unit 34. Since the finger pad T naturally touches the knob 110a, the left slanted surface 133a, the right slanted surface 133b, and the knob 110b in succession, the user can confirm such positions through tactile sensation, without requiring the user to look at the hand.

Because the knobs 110a, 110b and the pad 132 are located in adjacent positions, the working unit 12 can be both rolled and yawed in a composite motion using a single thumb. For example, if the working unit 12 is to be yawed to the left and rolled clockwise, the user places the thumb on both the left slanted surface 133a and the knob 110a, while depressing the left slanted surface 133a and pushing the knob 110a upwardly. If the working unit 12 is to be yawed to the right and rolled clockwise, the user places the thumb on both the right slanted surface 133b and the knob 110b, while depressing the right slanted surface 133b and pulling the knob 110b downwardly.

Also, when such a composite motion is performed, the manipulator is easy to operate, owing to the presence of a dead zone in each motion.

The manipulator 10 can be positioned easily and fixed with respect to an affected area of the patient. Further, for many types of surgical cases, the manipulator 10 can be prepared in a short period of time before each surgical operation, can be positioned flexibly for each surgical case, and does not pose a burden when drawing up operation protocols. Even if an unexpected trouble happens during a surgical operation when using the manipulator 10, for example, if the operation needs to be changed to a thoracotomy or a laparotomy, removal of the manipulator does not consume essential time so that the period of the operation is not unduly increased, resulting in a procedure which is highly non-invasive to the patient. Moreover, the manipulator 10 is simpler in structure and less expensive to manufacture than a remote control system.

In the manipulator 10 as described above, the turn-detecting first pressure-sensitive sensor 144a and the turn-detecting second pressure-sensitive sensor 144b are arranged such that the dead zone for operation of the shuttle ring 100 is obtained with reference to the neutral position. Also, the tilt-detecting first pressure-sensitive sensor 146a and the tilt-detecting second pressure-sensitive sensor 146b are arranged such that the dead zone for operation of the pad 132 is obtained with reference to the neutral position. With the above arrangement, there is play in each motion, and such play prevents a useless motion of the working unit 12 and an excessive reaction of the working unit 12 by slightly touching the input unit, thereby obtaining a high operability.

Further, the working unit 12 is moved to the right and left in a yawing direction and is turned clockwise and counterclockwise in response to the right and left operations and the clockwise and counterclockwise operations of the input unit with reference to the neutral position, respectively, thereby enabling a user to operate the manipulator intuitively.

In the manipulator 10, since the shuttle ring 100 and the pad 132 are arranged concentratedly on the composite input unit 34, the shuttle ring 100 and the pad 132 can be operated by moving the thumb within a small range. Further, the complex motion of the shuttle ring 100 and the pad 132 can be easily performed by one finger.

In the composite input unit 34, the rolling motion of the shuttle ring 100 corresponds to the rolling motion of the working unit 12, and the tilting motion of the pad 132 corresponds to the tilting motion of the working unit 12 in the yaw axis. Thus, the user can operate the working unit 12 easily and intuitively.

The shuttle ring 100 surrounds the pad 132 entirely, and the shuttle ring 100 is disposed in a well-balanced fashion, in coaxial relation to the center of the pad 132 (the axis J). The shuttle ring 100 and the pad 132 thus can be operated highly effectively and smoothly, allowing the user easily to learn how to use them. The effect of the well-balanced fashion is due to the surrounding of the pad 132 by the shuttle ring 100 at least with respect to the tilting directions of the pad 132.

In the composite input unit 34, the user can easily confirm the position of the shuttle ring 100 and the pad 132 through tactile sensation, without requiring the user to look at the hand and can operate them highly effectively and smoothly. Thus, the above composite input unit may appropriately be applied to perform fine techniques for a long time.

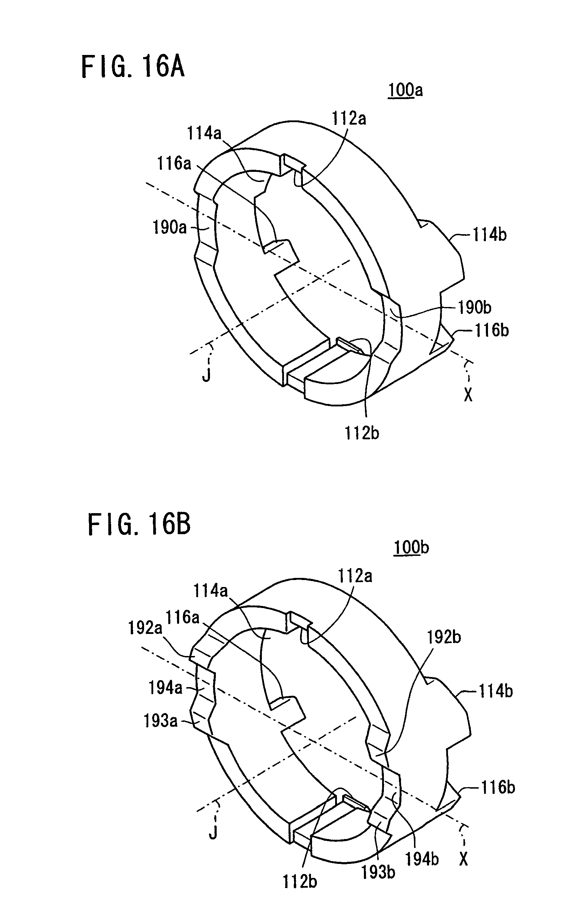

Modifications to the manipulator 10 and parts thereof shall be described below with reference to FIGS. 16A through 23. Parts thereof that are identical to those of the manipulator 10 are denoted using identical reference characters, and will not be described in detail below.

FIGS. 16A through 17 shows shuttle rings 100a, 100b, 100c, which make up input means according to respective modifications of the shuttle ring 100. Operation of the shuttle rings 100a, 100b, 100c is equally favorable to that of the shuttle ring 100. Also, in the shuttle rings 100a through 100c, a dead zone with reference to a neutral position is provided, as with the shuttle ring 100.

The shuttle ring 100a shown in FIG. 16A includes a pair of recesses (finger holders) 190a, 190b instead of the knobs 110a, 110b. The recesses 190a, 190b are slightly dented in the J2 direction for receiving and being pressed by the finger pad T of the thumb. For example, each of the recesses 190a, 190b may have a depth within a range of from 1 mm to 5 mm, and a circumferential length within a range of from 3 mm to 10 mm. The recesses 190a, 190b are disposed in symmetrical positions, which are spaced from each other diametrically across the axis J.

The shuttle ring 100b shown in FIG. 16B includes a pair of upper and lower small knobs 192a, 193a instead of the knob 110a, and a pair of upper and lower small knobs 192b, 193b instead of the knob 110b. The upper and lower small knobs 192a, 193a are disposed in vertically symmetrical positions, with a recess (finger presser) 194a defined therebetween. Similarly, the upper and lower small knobs 192b, 193b are disposed in vertically symmetrical positions, with a recess (finger presser) 194b defined therebetween.

The recesses 194a, 194b are slightly dented in the J2 direction for receiving and being pressed by the finger pad T of the thumb. For example, the recesses 194a, 194b may be positioned and shaped similarly to the recesses 190a, 190b.

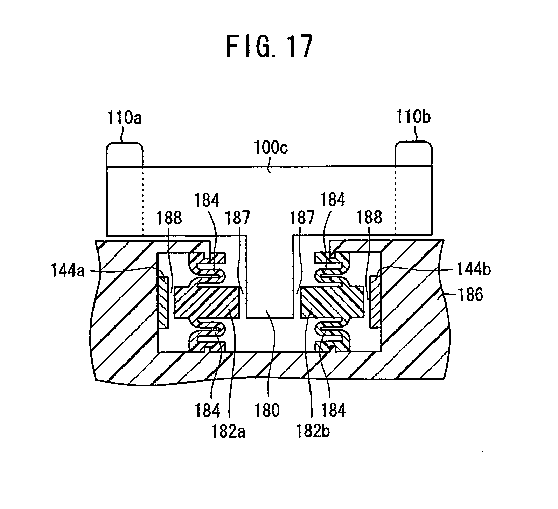

The shuttle ring 100c shown in FIG. 17 includes a single slender protrusion 180 instead of the protrusions 114a, 114b. The protrusion 180 is positioned between left and right rubber members 182a, 182b, which are joined by a folded web 184 to a base 186 of the grip handle 26. The rubber members 182a, 182b are elastically deformable circumferentially upon flexure of the folded web 184.

The turn-detecting first pressure-sensitive sensor 144a and the turn-detecting second pressure-sensitive sensor 144b are disposed across the rubber members 182a, 182b from the protrusion 180. In the neutral position, narrow gaps 187 are provided between the protrusion 180 and the rubber members 182a, 182b, and further, narrow gaps 188 are provided between the rubber members 182a, 182b and the turn-detecting first and second pressure-sensitive sensors 144a, 144b.

When the shuttle ring 100c is turned clockwise, the protrusion 180 is displaced to the left, as shown in FIG. 17, causing the rubber member 182a to press the turn-detecting first pressure-sensitive sensor 144a, which detects a clockwise angular displacement of the shuttle ring 100c. When the shuttle ring 100c is turned counterclockwise, the protrusion 180 is displaced to the right, as shown in FIG. 17, causing the rubber member 182b to press the turn-detecting second pressure-sensitive sensor 144b, which detects a counterclockwise angular displacement of the shuttle ring 100c. The gaps 187, 188 provide a dead zone during movement of the shuttle ring 100c, for thereby preventing the working unit 12 from being operated against the will of the user. The dead zone provides a certain amount of play, which allows the user to operate the shuttle ring 100c easily.

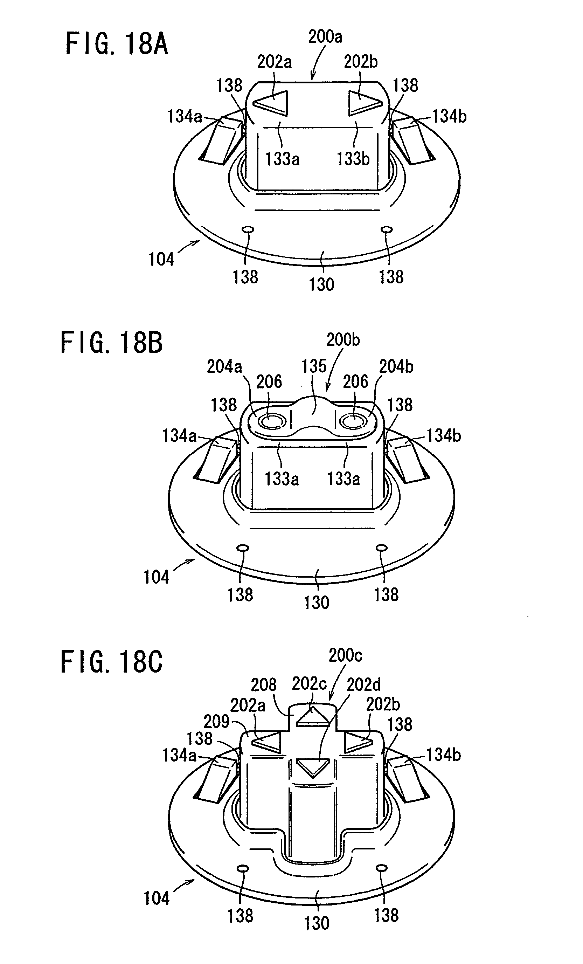

FIGS. 18A through 18C show pads 200a, 200b, 200c, forming input means according to respective modifications of the pad 132. Operability of the pads 200a, 200b, 200c is equally favorable to that of the pad 132.

As shown in FIG. 18A, the pad 200a does not contain the boss 135, but includes outwardly-pointed triangular lands 202a, 202b, disposed respectively on left and right slanted surfaces 133a, 133b. Even in the absence of the boss 135, the triangular lands 202a, 202b allow the user to recognize the positions of the left and right slanted surfaces 133a, 133b through tactile sensation. The user can also recognize the orientations indicated by the triangular lands 202a, 202b, through visual or tactile sensation, so as to recognize the yawing direction in which the working unit 12 is tilted.

As shown in FIG. 18B, the pad 200b includes a roof-shaped boss 135, which is smoothly joined to a left semicircular plate 204a placed on the left slanted surface 133a, and to a right semicircular plate 204b placed on the right slanted surface 133b. The semicircular plates 204a, 204b have respective shallow gradual recesses 206 defined therein. The roof-shaped boss 135 of the pad 200b allows the user to distinguish between the left and right slanted surfaces 133a and 133b. The semicircular plates 204a, 204b also allow the user to recognize the positions of the left and right slanted surfaces 133a and 133b. Because of the recesses 206, the user can easily apply the thumb to the semicircular plates 204a, 204b, thereby improving operability of the pad 200b.

As shown in FIG. 18C, the pad 200c comprises a combination made up of the pad 200a shown in FIG. 17A, which acts as a horizontal switch 209, together with a vertical switch 208 added thereto. The vertical switch 208 and the horizontal switch 209 are jointly provided in a crisscross shape. The switch 208 includes outwardly-pointed triangular lands 202c, 202d disposed on upper and lower portions thereof, respectively.

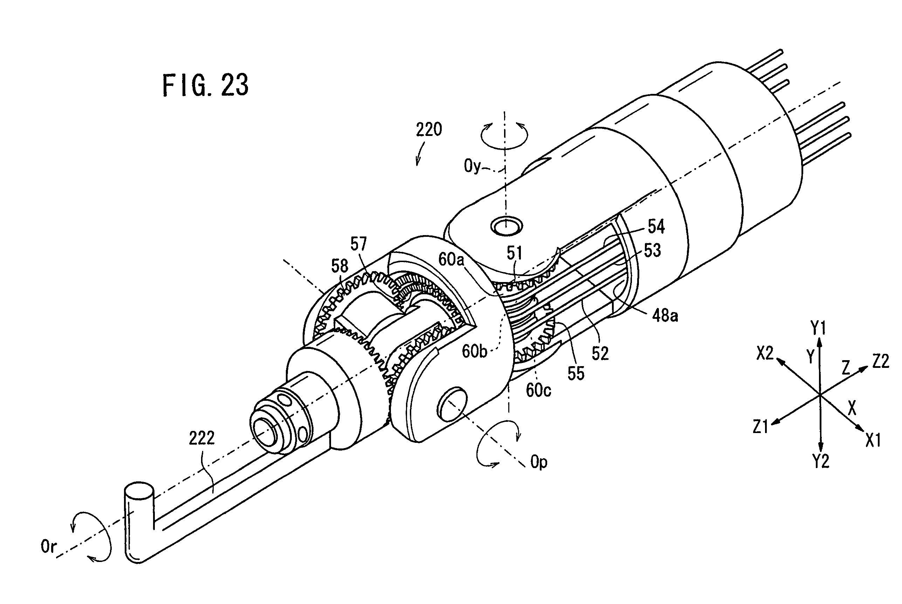

The pad 200c permits entry of commands through the composite input unit 34 in a concentrated manner, wherein such commands are supplied to a mechanism for operating the working unit 12 in directions other than yawing directions, e.g., for opening and closing the gripper 59, or for moving the working unit 12 in pitching directions. If the pad 200c is incorporated in a working unit 220 (see FIG. 23), which includes an electrosurgical knife as described later, wherein the horizontal switch 209 is assigned to yaw axis movements and the vertical switch 208 is assigned to pitch axis movements thereof, then the directions in which the switches extend and the directions in which the switches are pushed correspond to the directions in which the yaw and pitch axis movements are made. Therefore, the pad 200c allows a user to operate the working unit 12 intuitively.

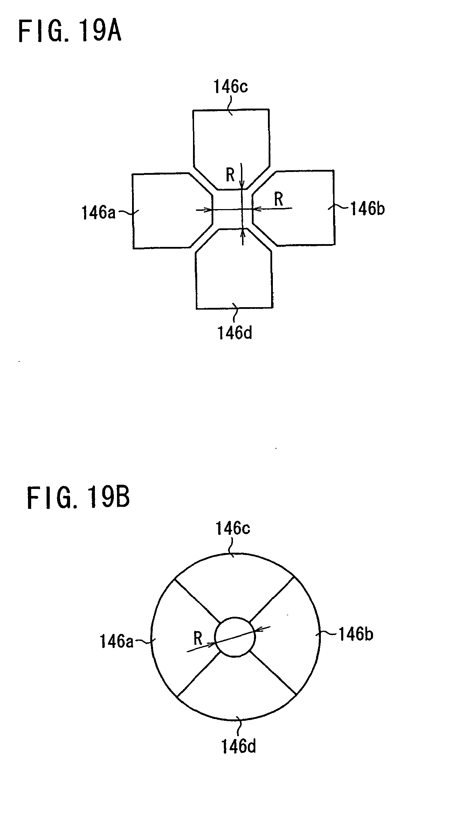

As shown in FIG. 19A, the tilt-detecting first pressure-sensitive sensor 146a and the tilt-detecting second pressure-sensitive sensor 146b are positioned beneath the horizontal switch 209, while a tilt-detecting third pressure-sensitive sensor 146c and a tilt-detecting fourth pressure-sensitive sensor 146d are positioned beneath the vertical switch 208. The pad 200c has an end surface 139 in the J2 direction, which has a crisscross shape. When the pad 200c is tilted in vertical (opposite) directions, the tilting and/or pressing forces applied thereto are detected by the tilt-detecting third pressure-sensitive sensor 146c and the tilt-detecting fourth pressure-sensitive sensor 146d.

As described above, the tilt-detecting first pressure-sensitive sensor 146a and the tilt-detecting second pressure-sensitive sensor 146b are horizontally spaced from each other by the distance R. Further, the tilt-detecting third pressure-sensitive sensor 146c and the tilt-detecting fourth pressure-sensitive sensor 146d are vertically spaced from each other by the distance R. Therefore, an appropriate dead zone is provided within the vertical tilting movements of the pad 200c.

Alternatively, as shown in FIG. 19B, the tilt-detecting first pressure-sensitive sensor 146a, the tilt-detecting second pressure-sensitive sensor 146b, the tilt-detecting third pressure-sensitive sensor 146c, and the tilt-detecting fourth pressure-sensitive sensor 146d may each have an arcuate shape of about 90°, wherein the sensors are disposed in left, right, upper, and lower positions, respectively, so as to jointly form an annular shape. Also, in the configuration shown in FIG. 18B, the end surface 139 of the pad in the J2 direction has a circular shape.

The annular shape, which is provided jointly by the tilt-detecting first pressure-sensitive sensor 146a, the tilt-detecting second pressure-sensitive sensor 146b, the tilt-detecting third pressure-sensitive sensor 146c and the tilt-detecting fourth pressure-sensitive sensor 146d, preferably has a diameter that is equal to the distance R. Therefore, an appropriate dead zone is established during each of horizontal and vertical tilting movements of the pad.

With the operating means shown in FIGS. 18C, 19A and 19B, the pad 200c is tilted in horizontally pushed directions in order to supply operation commands to a first rotational axis, e.g., a pivot axis. Further, the pad 200c is tilted in vertically pushed directions in order to supply operation commands to a second rotational axis, e.g., the gripper 59.

Also, the tilt-detecting first pressure-sensitive sensor 146a and the tilt-detecting second pressure-sensitive sensor 146b are a set of sensors for detecting right and left tilts, and are defined as a first set of sensors. These sensors face right and left portions of the end surface 139 respectively and are arranged with gaps therebetween. Further, the tilt-detecting third pressure-sensitive sensor 146c and the tilt-detecting fourth pressure-sensitive sensor 146d are a set of sensors for detecting a vertical tilt, and are defined as a second set of sensors. These sensors face upper and lower portions of the end surface 139 respectively and are arranged with gaps therebetween.

The vertical switch 208 of the pad 200c is referred to as the third input member of the composite input unit 34. The vertical switch 208 is integrally combined with the horizontal switch 209 (referred to as the second input member) of the pad 200c. Therefore, the vertical switch 208 and the horizontal switch 209 can be operated effectively and smoothly.

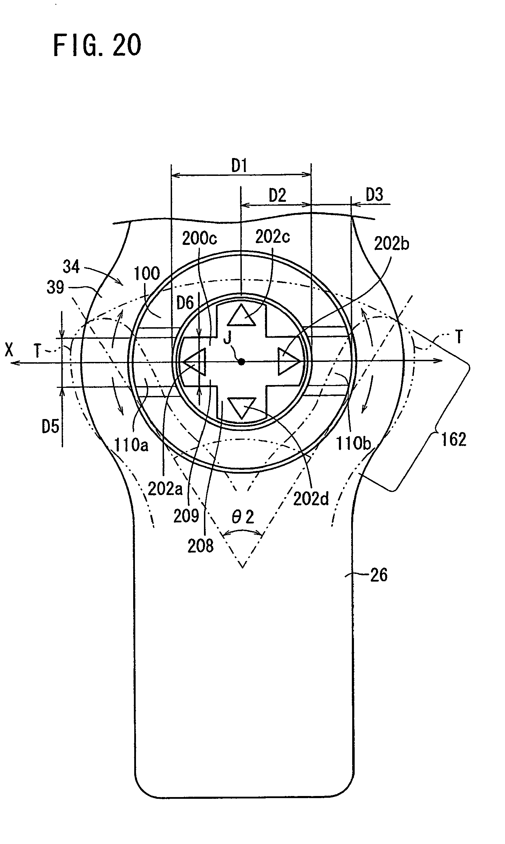

FIG. 20 shows the composite input unit 34 incorporating the pad 200c. In the composite input unit 34, when viewed in front elevation, the shuttle ring 100 forming the first input member surrounds the vertical switch 208 and the horizontal switch 209. The shuttle ring 100, the vertical switch 208, and the horizontal switch 209 are disposed in a well-balanced fashion, in coaxial relation to the axis J. The shuttle ring 100, the vertical switch 208, and the horizontal switch 209 thus can be operated highly effectively and smoothly, allowing the user easily to learn how to use them. If the vertical switch 208 is assigned to open and close the gripper 59, then since the gripper 59 is made up of a mechanism that can be opened and closed vertically while in a neutral position, the gripper 59 matches well with the vertical tilting movements of the vertical switch 208. Therefore, the pad 200c allows the user to operate the gripper 59 intuitively.

The layouts of the tilt-detecting first pressure-sensitive sensor 146a, the tilt-detecting second pressure-sensitive sensor 146b, the tilt-detecting third pressure-sensitive sensor 146c, and the tilt-detecting fourth pressure-sensitive sensor 146d, which are shown in FIGS. 19A and 19B, are effective to detect not only horizontal and vertical tilting movements of the pad 200c, but also tilting movements of the pad 200c in oblique directions. For example, if the pad 200c is tilted obliquely upwardly toward the left in FIGS. 19A and 19B, then a tilting and/or pressing force can be detected based on a vectorial sum of two signals output from the tilt-detecting first pressure-sensitive sensor 146a and the tilt-detecting third pressure-sensitive sensor 146c, and hence, the direction in which the pad 200c is tilted can be detected based on the direction of such a vectorial sum.

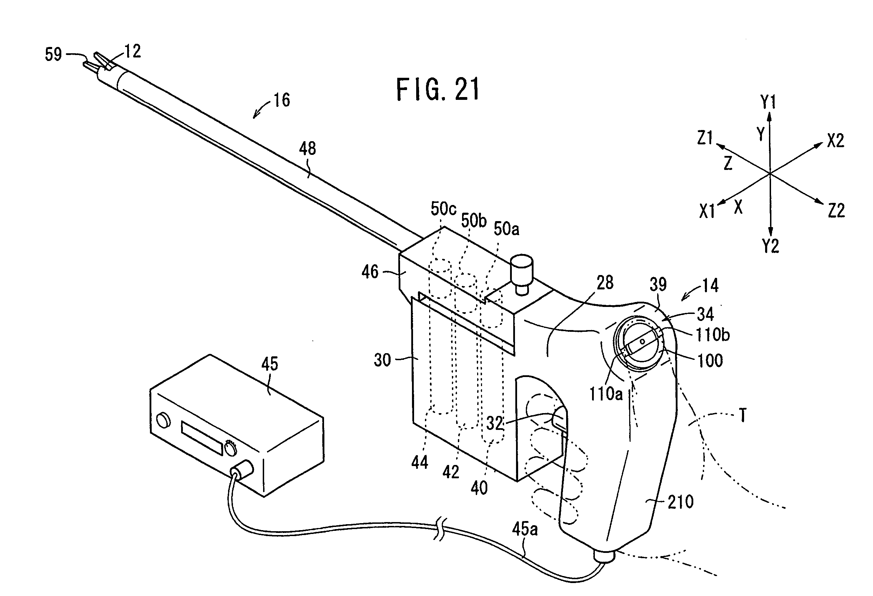

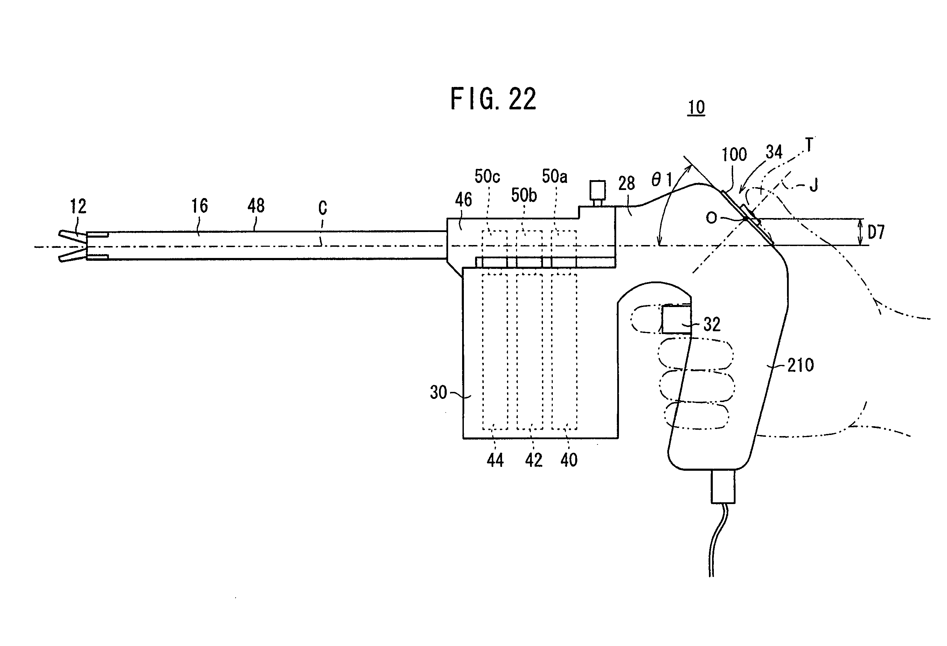

FIGS. 21 and 22 show a manipulator 10, including a grip handle 210 as a grip means, corresponding to the grip handle 26 referred to above. The grip handle 210 is disposed at a position that is shifted in the J1 direction from the grip handle 26. Stated otherwise, the center of rotation **O** of the knobs 110a, 110b is shifted upwardly from the longitudinal axis C of the connector shaft 48 in the Y1 direction, opposite to the Y2 direction in which the grip handle 26 extends. The center of rotation **O** is spaced from the axis C by a distance D7 of 30 mm.

Since the center of rotation **O** is shifted from the axis C, the composite input unit 34 also is disposed in a shifted position. The position at which the hand grips the grip handle 210 is also shifted in the Y1 direction, with the wrist being closer to the axis C. Therefore, when the manipulator 10 is turned about the trocar 20, the wrist is rotated about its own axis, rather than revolving around the axis C. Therefore, the manipulator 10 can easily be operated simply by twisting the wrist, and not by turning the wrist through a large arcuate path around the axis C. The distance D7 may be set to a value within a range of from 20 mm to 50 mm, for improving operability of the manipulator 10.

FIG. 23 shows a modified working unit 220, serving as a working means corresponding to the working unit 12 described above. The working unit 220 comprises an electrosurgical knife for passing an electric current through a living tissue at a high frequency, in order to coagulate, cut or treat a desired area of living tissue. The working unit 220 includes an electrode 222, which is positioned in place of the gripper 59 referred to above. The electrode 222 projects in the Z1 direction and has a tip end bent in the Y1 direction. The working unit 220 also includes a pitching mechanism, for turning the electrode 222 in pitching directions about an axis Op. Thus, the working unit 220 is capable of moving the electrode 222 in yawing, pitching, and rolling directions. When the manipulator 10 incorporates the working unit 220, the manipulator further employs a power supply 224 (see FIG. 1) for applying a voltage between the living tissue and the manipulator 10.

The composite input unit 34 may be applied and used together with the working unit 220. Specifically, the shuttle ring 100 is turned so as to turn the electrode 222 in rolling directions, and the vertical switch 208 of the pad 200c, shown in FIG. 20, is tilted so as to turn the electrode 222 in both yawing and pitching directions.

The composite input unit 34 may also be used with a working unit including a rolling mechanism. For example, the composite input unit 34 may be used with a working unit having a yaw axis, a roll axis and a gripper axis, a working unit having a yaw axis, a pitch axis, a roll axis and an electrosurgical knife electrode, or a working unit having a yaw axis, a pitch axis, a roll axis and a gripper axis. In the case of such working units, the gripper axis or the electrosurgical knife electrode should be positioned at the distal end, with the other axes thereof being arranged in any desired sequence. However, if the manipulator is used in a surgical operation for removal of the prostate gland, then the gripper axis should be positioned at the distal end, whereas the roll axis should be positioned as the second axis from the distal end.

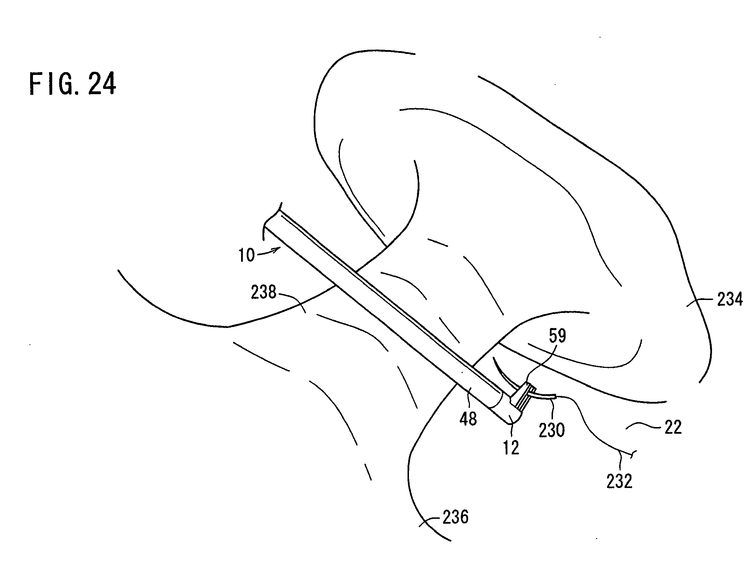

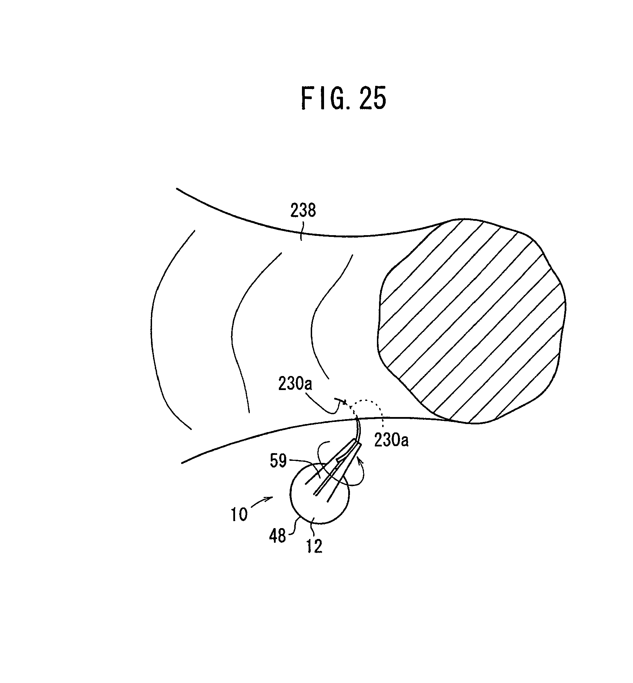

An endoscopic surgical operation for removing a prostate gland using the manipulator 10 shall be described below with reference to FIGS. 24 and 25.

FIG. 24 shows the manner in which the working unit 12 and the gripper 59 on the distal end of the connector shaft 48 are inserted to a position near a region to be treated in a body cavity 22, and thereafter the region is sutured and a suture knot is tied, in a process of peeling off and cutting the urethra during an operation to remove the prostate gland. The body cavity 22 into which the manipulator 10 is inserted accommodates therein a body cavity wall 234, a prostate gland 236, and a dorsal vein complex (DVC) 238, wherein the urethra is disposed in a rear part thereof. As can be seen in FIG. 24, the body cavity 22 is a narrow deep space.

Before the prostate gland 236 is cut off, it is necessary to tie a suture in the DVC 238 in order to arrest hemorrhage. For tying a suture in the DVC 238, a curved needle 230 with a suture 232 joined thereto must be passed through the rear part of the DVC 238 into the narrow deep body cavity 22. However, with a conventional forceps, it is quite difficult to perform such a technique.

To perform techniques with the manipulator 10 according to the process shown in FIG. 4, the working unit 12 is first inserted into the body cavity 22 through the trocar 20, and the working unit 12 and the gripper 59 are oriented upwardly. Specifically, as shown in FIG. 4, the pad 132 is operated so as to orient the gripper 59 to the right, and the grip handle 26 is held substantially horizontally so that the gripper 59 is directed substantially upwardly. Then, the curved needle 230 is gripped by the gripper 59, substantially perpendicularly to the gripper 59. After the working unit 12 has been inserted into the body cavity 22, the working unit 12 is positioned slightly rightward of the DVC 238, while the gripper 59 is held substantially parallel to the DVC 238.

Then, as shown in FIG. 25, the shuttle ring 100 is operated in order to turn the gripper 59 in a rolling direction, to thereby introduce the gripped curved needle 230 into the DVC 238. The gripper 59 is sufficiently turned until the tip end 230a of the curved needle 230 emerges from a face part of the DVC 238. Rolling movement of the gripper 59 is easily accomplished by pushing or pulling the knob 110a or 110b of the shuttle ring 100.

The above movement of the gripper 59 can primarily be accomplished simply by operating the shuttle ring 100. However, depending on the surgical case or situation, the gripper 59 may simultaneously be moved in yawing directions, and/or the connector shaft 48 may simultaneously be pushed or pulled, or moved vertically, in combination with the rolling movement of the gripper 59.

Thereafter, the gripper 59 is opened in order to release the curved needle 230, and then, the projecting tip end 230a of the curved needle 230 is gripped by the gripper 59 and pulled so as to remove the curved needle 230 through the DVC 238. At this point, a first piercing cycle is finished. Thereafter, successive piercing cycles are performed and the suture 232 is tied longitudinally along the DVC 238, in a plurality of cycles following the respective piercing cycles, thereby arresting hemorrhage in the blood vessel in the DVC 238.

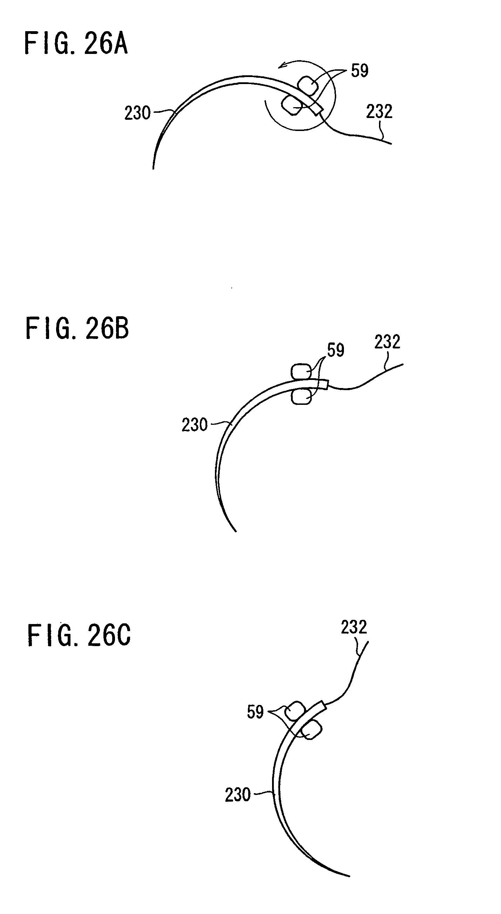

The manipulator 10 includes a mechanism for turning the gripper 59 on the tip end of the working unit 12, about an axis along which the gripper 59 extends, wherein the mechanism is rolled by the shuttle ring 100. As shown in FIGS. 26A, 26B, and 26C, irrespective of the direction in which the main shaft 62 (see FIG. 3) is oriented when turned around the first rotational axis Oy, the gripper 59 is turned about its own axis as viewed from the tip end thereof, to thereby turn the curved needle 230 with ease. Since the curved needle 230 is turned based on operation of the shuttle ring 100, the curved needle 230 can easily be passed through the rear part of the DVC 238 and easily removed from the face part of the DVC 238.

The manipulator 10 and the working units 12, 220 have been illustrated as being used in medical applications. However, the manipulator 10 and the working units 12, 220 can also be used in other applications, for example, in order to repair narrow regions of energy-related devices and apparatuses.

A manipulator (10) includes an operation command unit (14) to be gripped by a hand, a composite input unit (34) operable by a finger, a connector shaft (48) extending from the operation command unit, and a working unit (12) disposed on a distal end of the connector shaft. The working unit (12) includes a yawing mechanism, a rolling mechanism, and an opening and closing mechanism. The composite input unit (34) has a shuttle ring (100) for actuating the rolling mechanism, and a pad (132) for actuating the yawing mechanism. The shuttle ring (100) is an angularly movable member having two knobs (110a, 110b) disposed in respective left and right positions. The pad (132) is disposed inside of the shuttle ring (100).

## Claims

1. A manipulator comprising:
an operating unit (14) including a grip handle (26) to be gripped by a hand;
an input unit (132) mounted on said operating unit (14) and operable in either direction with reference to a neutral position;
a first detecting unit (146a) for detecting an operating amount of said input unit (132) in one direction of the directions;
a second detecting unit (146b) for detecting an operating amount of said input unit (132) in another direction of the directions;
a connector (48) extending from said operating unit (14); and
a working unit (12) disposed on a distal end of said connector (48) and movable in either direction based on a signal detected by said first detecting unit (146a) and said second detecting unit (146b),
**characterized in that**
said first detecting unit (146a) and said second detecting unit (146b) are arranged such that an operating amount of said input unit (132) is not detected within a range of a certain dead zone with reference to the neutral position,
said working unit (12) comprises a rotating mechanism angularly movable about an axis along which a distal end of said working unit (12) extends, based on operation of said input unit (132);
said input unit (132) comprises a rolling member (139,139a,139b) for supplying an operation command to said rotating mechanism; and
said first detecting unit (146a) and said second detecting unit (146b) are arranged such that an operating amount of said input unit (132) is not detected within said range of said certain dead zone ranging from ± 1° to ± 8° with reference to the neutral position.

## Patentansprüche

1. Manipulator mit:
einer Betätigungseinheit (14) mit einer Ergreifhandhabe (26), die durch eine Hand ergriffen wird;
einer Eingabeeinheit (132), die an der Betriebseinheit (14) montiert ist und in irgendeine Richtung unter Bezugnahme auf eine neutrale Position betreibbar ist;
einer ersten Erfassungseinheit (146a) zum Erfassen eines Betätigungsbetrages der Eingabeeinheit (132) in einer Richtung der Richtungen;
einer zweiten Erfassungseinheit (146b) zum Erfassen eines Betätigungsbetrages der Eingabeeinheit (132) in einer anderen Richtung der Richtungen;
einer Verbindungseinrichtung (48), die sich von der Betriebseinheit (14) erstreckt; und
einer Arbeitseinheit (12), die an einem distalen Ende der Verbindungseinrichtung (48) angeordnet ist und in irgendeine Richtung auf der Grundlage eines Signals bewegbar ist, das durch die erste Erfassungseinheit (146a) und die zweite Erfassungseinheit (146b) erfasst wird,
**dadurch gekennzeichnet, dass**
die erste Erfassungseinheit (146a) und die zweite Erfassungseinheit (146b) derart angeordnet sind, dass ein Betätigungsbetrag der Eingabeeinheit (132) nicht innerhalb eines Bereiches einer bestimmten Totzone in Bezug auf die neutrale Position erfasst wird,
wobei die Arbeitseinheit (12) einen Drehmechanismus aufweist, der um eine Achse, entlang der sich ein distales Ende der Arbeitseinheit (12) erstreckt, auf der Grundlage der Betätigung der Eingabeeinheit (132) winklig bewegbar ist;
wobei die Eingabeeinheit (132) ein Rollelement (139, 139a, 139b) aufweist für ein Liefern eines Betätigungsbefehls zu dem Drehmechanismus; und
die erste Erfassungseinheit (146a) und die zweite Erfassungseinheit (146b) derart angeordnet sind, dass ein Betätigungsbetrag der Eingabeeinheit (132) innerhalb des Bereiches der bestimmten Totzone nicht erfasst wird, der von ± 1° bis ± 8° in Bezug auf die neutrale Position reicht.

## Revendications

1. Manipulateur comprenant :
une unité d'opération (14) comprenant une poignée de préhension (26) à tenir à la main ;
une unité d'entrée (132) montée sur ladite unité d'opération (14) et pouvant agir dans n'importe quelle direction par rapport à une position neutre ;
une première unité de détection (146a) permettant de détecter une quantité opérante de ladite unité d'entrée (132) dans une direction desdites directions ;
une deuxième unité de détection (146b) permettant de détecter une quantité opérante de ladite unité d'entrée (132) dans une autre direction desdites directions ;
un connecteur (48) s'étendant à partir de ladite unité opérante (14) ; et
une unité de travail (12) disposée sur une extrémité distale dudit connecteur (48) et mobile dans une direction quelconque en fonction d'un signal détecté par ladite première unité de détection (146a) et par ladite deuxième unité de détection (146b),
**caractérisé en ce que**
ladite première unité de détection (146a) et ladite deuxième unité de détection (146b) sont disposées de sorte qu'une quantité opérante de ladite unité d'entrée (132) n'est pas détectée dans un intervalle d'une certaine zone morte en lien avec la position neutre,
ladite unité de travail (12) comprend un mécanisme tournant mobile de manière angulaire autour d'un axe, le long duquel s'étend une extrémité distale de ladite unité de travail (12), selon le fonctionnement de ladite unité d'entrée (132) ;
ladite unité d'entrée (132) comprend un élément roulant (139, 139a, 139b) permettant de transmettre une commande d'opération audit mécanisme tournant ; et
ladite première unité de détection (146a) et ladite deuxième unité de détection (146b) sont disposées de sorte qu'une quantité opérante de ladite unité d'entrée (132) n'est pas détectée dans ledit intervalle de ladite certaine zone morte, comprise entre ± 1° et ±8°, en lien avec la position neutre.
